(19) Europäisches Patentamt
European Patent Office
Office européen des brevets

(11) **EP 4 481 374 A1**

(12) **EUROPEAN PATENT APPLICATION**
published in accordance with Art. 153(4) EPC

(43) Date of publication:
**25.12.2024 Bulletin 2024/52**

(21) Application number: **22926338.9**

(22) Date of filing: **29.11.2022**

(51) International Patent Classification (IPC):
***G01N 27/26*** (2006.01)        ***G01N 27/416*** (2006.01)

(52) Cooperative Patent Classification (CPC):
**G01N 27/26; G01N 27/416**

(86) International application number:
**PCT/JP2022/043871**

(87) International publication number:
**WO 2023/157421 (24.08.2023 Gazette 2023/34)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **18.02.2022 JP 2022023685**

(71) Applicant: **HITACHI HIGH-TECH CORPORATION
Tokyo 105-6409 (JP)**

(72) Inventor: **YAMASHITA Kotaro
Tokyo 105-6409 (JP)**

(74) Representative: **MERH-IP Matias Erny Reichl
Hoffmann
Patentanwälte PartG mbB
Paul-Heyse-Straße 29
80336 München (DE)**

(54) **ELECTROLYTE CONCENTRATION MEASURING DEVICE AND METHOD FOR OBTAINING SELECTIVITY COEFFICIENT**

(57)    It is possible to obtain the selectivity coefficient of the interfering ions or the value corresponding to the concentration of the interfering ions, without the need for a dedicated sample for calculating the selectivity coefficient of the interfering ions. An electrolyte concentration measuring device 100 that measures an ion concentration of analyte ions contained in a liquid, and the electrolyte concentration measuring device includes: an ion selective electrode 111 to 113 to which the liquid is supplied; a reference electrode 116 serving as a reference for the potential; and a control unit 120 configured to output the ion concentration of the analyte ions based on an electromotive force between the reference electrode 116 and the ion selective electrode 111 to 113, in which the control unit 120 obtains a selectivity coefficient of interfering ions that affect measurement of a concentration of the analyte ions or a value corresponding to a concentration of the interfering ions from a trained model by inputting a measured potential of a solution containing the analyte ions and the interfering ions into the trained model, the trained model being configured to output the selectivity coefficient of the interfering ions or the value corresponding to the concentration of the interfering ions based on the measured potential of the solution containing the analyte ions and the interfering ions.

[FIG. 2]

EP 4 481 374 A1

## Description

Technical Field

The present disclosure relates to an electrolyte concentration measuring device and a method for obtaining a selectivity coefficient, and particularly relates to an electrolyte concentration measuring device and a method for obtaining a selectivity coefficient capable of calculating selectivity of interfering ions.

Background Art

Since an ion selective electrode can selectively quantify a concentration of specific ions in a liquid, the ion selective electrode is used in a wide field such as a water quality analysis and a medical field. In the medical field in particular, since there is a close relationship between a metabolic reaction of a living body and an ion concentration, high blood pressure symptoms, kidney disease, neurological disorder, and the like can be diagnosed by quantifying specific ions contained in a biological sample such as blood and urine. Since it is necessary to continuously analyze a large number of specimens in clinical examination, a high-throughput electrolyte concentration measuring device equipped with the ion selective electrode is used on a daily basis.

The electrolyte concentration measuring device mainly measures a concentration of cations such as sodium ions and potassium ions and a concentration of anions such as chloride ions as measurement items. In relation to cations, neutral carrier molecules that selectively capture specific cations such as crown ethers and valinomycin and that have no charge are found. A membrane containing the neutral carrier molecules is generally used as an ion-sensitive membrane of a cation selective electrode for sodium, potassium, or the like, and the ion-sensitive membrane has high ion selectivity. On the other hand, in relation to anions, there is no neutral carrier suitable for an ion selective electrode of an electrolyte concentration measuring device that requires quick response of high throughput, and an ion-sensitive membrane having various configurations is used.

In the case of an anion selective electrode containing a quaternary ammonium salt as a ligand, selectivity is determined in an order reverse to an order (the following inequation) that is called an elution permutation depending on a degree of hydration of ions.

$$SCN^- < I^- < ClO_3^- < NO_3^- < Br^- < Cl^- < CH_3COO^- < SO_4^{2-} < tartaric\ acid < citric\ acid$$

When a chloride ion concentration in a biological sample such as serum or plasma is measured, interfering ions other than ions to be measured are often contained in the biological sample. In general, blood of a human body contains bicarbonate ions ($HCO_3^-$) having a chloride ion concentration of about 25%, and the bicarbonate ions ($HCO_3^-$) have the highest concentration among interfering ions. For example, in measurement of the chloride ions, quality of selectivity to the bicarbonate ions affects a measured value of the chloride ion concentration. A change over time in selectivity of an anion selective electrode for the bicarbonate ions, an abnormality of an ion selective electrode, and the like affect a measured value of the chloride ion concentration.

PTL 1 discloses a technique related to an automatic analyzer that calculates a concentration of analyte ions contained in a sample by using a result of calculating a selectivity coefficient and a result of measuring a concentration of coexisting ions contained in the sample without adding an ion selective electrode other than an ion selective electrode.

Citation List

Patent Literature

PTL 1: WO2019/163281

Summary of Invention

Technical Problem

In PTL 1, in order to calculate the selectivity coefficient, a selectivity coefficient calculation sample 13 in which a concentration of sodium ions and the concentration of the coexisting ions (interfering ions) are known is measured. Specifically, in PTL 1, two selectivity coefficient calculation samples 13 having different interfering ion concentrations are measured, and a selectivity coefficient of the interfering ions is calculated based on a measured result.

However, in PTL 1, it is necessary to prepare a dedicated selectivity coefficient calculation sample for calculating the selectivity coefficient of the interfering ions and it is necessary to measure the selectivity coefficient calculation sample.

The present disclosure provides an electrolyte concentration measuring device and a method for obtaining a selectivity coefficient capable of obtaining a selectivity coefficient of interfering ions or a value corresponding to a concentration of the interfering ions, without the need for a dedicated sample for calculating the selectivity coefficient of the interfering ions.

Solution to Problem

An electrolyte concentration measuring device according to the present disclosure is an electrolyte concentration measuring device that measures an ion concentration of analyte ions contained in a liquid, and the electrolyte concentration measuring device includes: an ion selective electrode configured to react with the analyte ions and output a potential corresponding to the ion concentration; a reference electrode configured to output a reference potential serving as a reference for the potential; and a control unit configured to output the ion concentration of the analyte ions based on a potential difference between the reference electrode and the ion selective electrode, in which the control unit obtains a selectivity coefficient of interfering ions that affect measurement of a concentration of the analyte ions or a value corresponding to a concentration of the interfering ions from a calculation unit by inputting a measured potential of a solution containing the analyte ions and the interfering ions into the calculation unit, the calculation unit being configured to output the selectivity coefficient of the interfering ions or the value corresponding to the concentration of the interfering ions based on the measured potential of the solution containing the analyte ions and the interfering ions.

Advantageous Effects of Invention

According to the present disclosure, it is possible to obtain the selectivity coefficient of the interfering ions or the value corresponding to the concentration of the interfering ions, without the need for a dedicated sample for calculating the selectivity coefficient of the interfering ions.

Brief Description of Drawings

[FIG. 1A] FIG. 1A is a schematic configuration diagram showing an electrolyte concentration measuring device according to Embodiment 1.
[FIG. 1B] FIG. 1B is a hardware block diagram showing a computer system according to Embodiment 1.
[FIG. 2] FIG. 2 is a schematic diagram showing a step (a learning phase) of constructing a final model and a step (a prediction phase) of predicting an unknown result using the constructed final model according to Embodiment 1.
[FIG. 3] FIG. 3 is a flowchart showing a flow of training an initial model.
[FIG. 4] FIG. 4 is a diagram showing a plurality of learning data sets.
[FIG. 5] FIG. 5 is a diagram showing plots of test data and predicted values of a model in which machine learning is performed by applying multiple regression to an algorithm using the data sets shown in FIG. 4.
[FIG. 6] FIG. 6 is a diagram showing plots of test data and predicted values of a model in which machine learning is performed by applying stochastic gradient descent regression to an algorithm using the data sets shown in FIG. 4.
[FIG. 7] FIG. 7 is a diagram showing plots of test data and predicted values of a model in which machine learning is performed by applying polynomial regression to an algorithm using the data sets shown in FIG. 4.
[FIG. 8] FIG. 8 is a diagram showing plots of test data and predicted values of a model in which machine learning is performed by applying Ridge regression to an algorithm using the data sets shown in FIG. 4.
[FIG. 9] FIG. 9 is a diagram showing plots of test data and predicted values of a model in which machine learning is performed by applying Lasso regression to an algorithm using the data sets shown in FIG. 4.
[FIG. 10] FIG. 10 is a diagram showing plots of test data and predicted values of a model in which machine learning is performed by applying Elastic Net regression to an algorithm using the data sets shown in FIG. 4.
[FIG. 11] FIG. 11 is a diagram showing plots of test data and predicted values of a model in which machine learning is performed by applying support vector regression to an algorithm using the data sets shown in FIG. 4.
[FIG. 12] FIG. 12 is a diagram showing plots of test data and predicted values of a model in which machine learning is performed by applying Support vector regression with Gaussian kernel to an algorithm using the data sets shown in FIG. 4.
[FIG. 13] FIG. 13 is a diagram showing plots of test data and predicted values of a model in which machine learning is performed by applying a decision tree to an algorithm using the data sets shown in FIG. 4.
[FIG. 14] FIG. 14 is a diagram showing plots of test data and predicted values of a model in which machine learning is performed by applying a random forest to an algorithm using the data sets shown in FIG. 4.
[FIG. 15] FIG. 15 is a diagram showing plots of test data and predicted values of a model in which machine learning is performed by applying XGBoost to an algorithm using the data sets shown in FIG. 4.

[FIG. 16] FIG. 16 is a diagram showing plots of test data and predicted values of a model in which machine learning is performed by applying a multilayer perceptron to an algorithm using the data sets shown in FIG. 4.

[FIG. 17] FIG. 17 is a flowchart showing deterioration determination processing according to Embodiment 1.

[FIG. 18] FIG. 18 is a flowchart showing maintenance support processing corresponding to a membrane fouling alarm according to Embodiment 1.

[FIG. 19] FIG. 19 is a flowchart showing processing of correcting a measured value of a control sample according to Embodiment 2.

Description of Embodiments

Embodiments of the present invention will be described in detail with reference to the drawings. In the following embodiments, it is needless to mention that components (also including element steps and the like) thereof are not necessarily essential unless otherwise specified or unless clearly considered to be essential in principle.

<Embodiment 1>

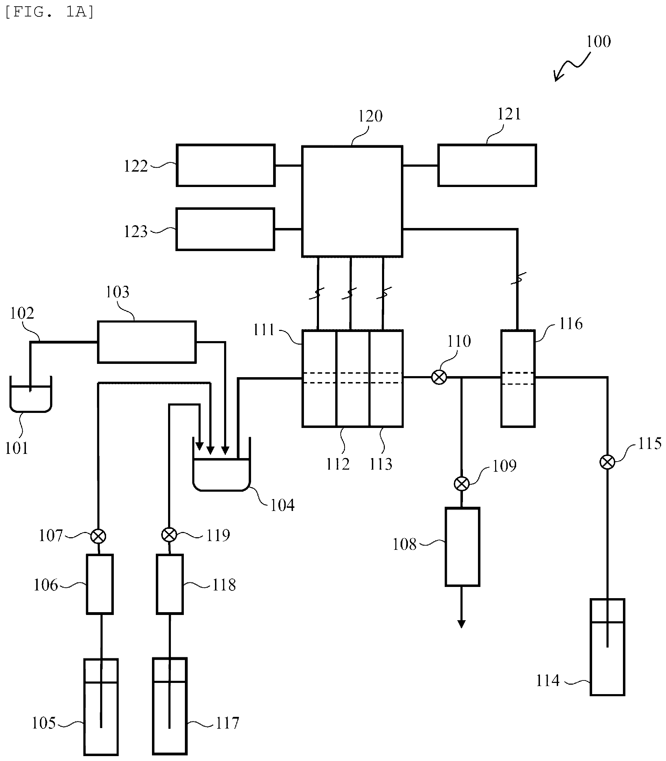

FIG. 1A is a schematic configuration diagram showing an electrolyte concentration measuring device according to Embodiment 1. An electrolyte concentration measuring device 100 according to Embodiment 1 is a device that measures a concentration of analyte ions contained in a liquid sample. Hereinafter, a configuration of the electrolyte concentration measuring device 100 will be described.

(Electrolyte Concentration Measuring Device 100)

A sample container 101 stores a biological sample (hereinafter, referred to as a sample) such as blood or urine. A sample aliquoting nozzle 102 is immersed in the sample stored in the sample container 101. The sample aliquoting nozzle 102 aspirates the sample from the sample container 101 by a set amount and discharges the sample to a diluent tank 104 by an operation of a sample aliquoting nozzle syringe 103. A diluent bottle 105 stores a diluent used for diluting the sample. The diluent is fed to the diluent tank 104 by operations of a diluent syringe 106 and a diluent solenoid valve 107. The sample in the diluent tank 104 is diluted with the diluent.

The diluted sample in the diluent tank 104 is supplied to a sodium ion selective electrode 111, a potassium ion selective electrode 112, and a chloride ion selective electrode 113 by operations of a sipper syringe 108, a sipper syringe solenoid valve 109, and a pinch valve 110. The sodium ion selective electrode 111 reacts with sodium ions and outputs a potential corresponding to a concentration of the sodium ions. The potassium ion selective electrode 112 reacts with potassium ions and outputs a potential corresponding to a concentration of the potassium ions. The chloride ion selective electrode 113 reacts with chloride ions and outputs a potential corresponding to a concentration of the chloride ions. A reference electrolyte stored in a reference electrolyte bottle 114 is supplied to a reference electrode 116 by operations of a reference electrolyte solenoid valve 115, the sipper syringe 108, and the sipper syringe solenoid valve 109. The reference electrode 116 outputs a reference potential serving as a reference for a potential output by each ion selective electrode. A control unit 120 obtains an electromotive force (a potential difference) between the reference electrode 116 and each of the ion selective electrodes 111, 112, and 113 to which the diluted sample is supplied. Hereinafter, when an electromotive force is simply described, it refers to the electromotive force between the reference electrode 116 and each of the ion selective electrodes 111, 112, and 113.

In measurement of an internal standard solution used for obtaining a concentration of the sample, an internal standard solution stored in an internal standard solution bottle 117 is fed, by operations of an internal standard solution syringe 118 and an internal standard solution solenoid valve 119, to the diluent tank 104 from which the sample and the diluent are discharged. The internal standard solution in the diluent tank 104 is supplied to the sodium ion selective electrode 111, the potassium ion selective electrode 112, and the chloride ion selective electrode 113 by operations of the sipper syringe 108, the sipper syringe solenoid valve 109, and the pinch valve 110. The reference electrolyte stored in the reference electrolyte bottle 114 is supplied to the reference electrode 116 by operations of the reference electrolyte solenoid valve 115, the sipper syringe 108, and the sipper syringe solenoid valve 109. The control unit 120 obtains an electromotive force between the reference electrode 116 and each of the ion selective electrodes 111, 112, and 113 to which the internal standard solution is supplied.

The sodium ion selective electrode 111, the potassium ion selective electrode 112, the chloride ion selective electrode 113, and the reference electrode 116 are connected to the control unit 120. The control unit 120 controls overall operations of the electrolyte concentration measuring device 100, obtains an electromotive force, and controls operations of the syringes 103, 106, 108, and 118, the solenoid valves 107, 109, 115, and 119, and the like. A storage unit 121, a display unit 122, and an input unit 123 are connected to the control unit 120. A user inputs various parameters and information of a measurement target sample (sample type information and the like) via the input unit 123 based on a setting screen or the like displayed on the display unit 122. The storage unit 121 stores the input information. In addition, the storage unit 121 stores various programs used in measurement of the sample, measured results, and the like. The storage unit 121 stores a trained model that outputs a selectivity coefficient of interfering ions or a value corresponding to a concentration of the interfering ions based on a measured potential of a solution containing analyte ions and the interfering ions that is measured by calibration to be described later. That is, the trained model functions as a calculation unit that outputs the selectivity coefficient of the interfering ions or the value corresponding to the concentration of the interfering ions based on the measured potential of the solution containing the analyte ions and the interfering ions.

(Computer System)

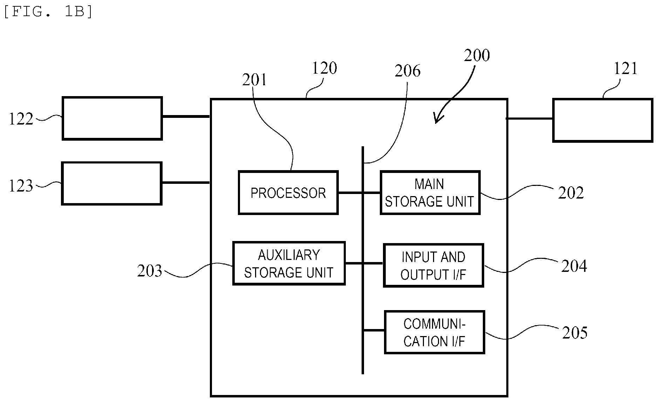

FIG. 1B is a hardware block diagram showing a computer system of the control unit according to Embodiment 1. A computer system 200 of the control unit 120 executes processing in a flowchart to be described later. The computer system 200 includes a processor 201, a main storage unit 202, an auxiliary storage unit 203, an input and output interface (hereinafter, the interface is abbreviated as I/F) 204, a communication I/F 205, and a bus 206 that is communicably connected to the above modules.

The processor 201 is a central processing unit that controls an operation of each unit communicably connected to the control unit 120. The processor 201 is, for example, a central processing unit (CPU), a digital signal processor (DSP), or an application specific integrated circuit (ASIC). The processor 201 loads a program (for example, a deterioration determination program) stored in the storage unit 121 to a work area of the main storage unit 202 in an executable manner and executes the program. The main storage unit 202 stores a program to be executed by the processor 201, data to be processed by the processor, and the like. The main storage unit 202 is a flash memory, a random access memory (RAM), or the like. The auxiliary storage unit 203 is, for example, a read only memory (ROM), and stores a boot program and various setting values of the control unit 120. The storage unit 121 stores an OS, a deterioration determination processing program to be described later, and the like. The storage unit 121 is a silicon disk including a nonvolatile semiconductor memory (a flash memory, an erasable programmable ROM (EPROM)), a solid state drive device, a hard disk drive (HDD) device, and the like.

The input and output I/F 204 is communicably connected to an input device (for example, the input unit 123 or the storage unit 121) and an output device (for example, the display unit 122 or the storage unit 121). The communication I/F 205 is an interface for communicably connecting the electrolyte concentration measuring device 100 to an external device via a network.

(Calibration)

Hereinafter, a method of calculating a slope sensitivity SL by calibration will be described.

A standard solution (L) having a known low concentration (a concentration $C_L$) is aliquoted into the diluent tank 104, and then the diluent in the diluent bottle 105 is further aliquoted into the diluent tank 104 using the diluent syringe 106. In this manner, the standard solution (L) having the known low concentration is diluted with a set ratio. Next, the sipper syringe 108 is operated to introduce the diluted standard solution (L) having the known low concentration in the diluent tank 104 into flow paths of the ion selective electrodes 111 to 113. Further, a reference electrolyte is introduced from the reference electrolyte bottle 114 into a flow path of the reference electrode 116. Accordingly, the reference electrolyte and the diluted standard solution (L) having the known low concentration come into contact with each other. Thereafter, the control unit 120 measures a potential difference (an electromotive force $EMF_L$ of the standard solution (L) having the known low concentration) between the reference electrode 116 and each of the ion selective electrodes 111 to 113.

Next, the internal standard solution in the internal standard solution bottle 117 is newly aliquoted into the diluent tank 104 using the internal standard solution syringe 118. Next, the sipper syringe 108 is operated to introduce the internal standard solution in the diluent tank 104 into the flow paths of the ion selective electrodes 111 to 113. Further, the reference electrolyte is introduced from the reference electrolyte bottle 114 into the flow path of the reference electrode 116. Accordingly, similar to the case of the standard solution (L) having the known low concentration, the reference electrolyte and the internal standard solution come into contact with each other, and the control unit 120 measures a potential difference (an electromotive force $EMF_{IS}$ of the internal standard solution) between the reference electrode 116 and each of the ion selective electrodes 111 to 113.

Next, a standard solution (H) having a known high concentration ($C_H$) is aliquoted into the diluent tank 104, and then the diluent in the diluent bottle 105 is further aliquoted into the diluent tank 104 using the diluent syringe 106. In this manner, the standard solution (H) having the known high concentration is diluted with a set ratio. Next, the sipper syringe 108 is operated to introduce the diluted standard solution (H) having the known high concentration in the diluent tank 104 into the flow paths of the ion selective electrodes 111 to 113. Further, the reference electrolyte is introduced from the reference electrolyte bottle 114 into the flow path of the reference electrode 116. Accordingly, the reference electrolyte and the diluted standard solution (H) having the known high concentration come into contact with each other. Thereafter, the control unit 120 measures a potential difference (an electromotive force $EMF_H$ of the standard solution (H) having the known high concentration) between the reference electrode 116 and each of the ion selective electrodes 111 to 113.

A slope sensitivity SL of a calibration curve is calculated using the following equation (1) based on the electromotive forces $EMF_H$ and $EMF_L$ measured by the control unit 120 as described above.

$$SL = (EMF_H - EMF_L)/(LogC_H - LogC_L) \quad \text{Equation (1)}$$

Calibration is executed by the above processing.

The slope sensitivity SL corresponds to $2.303 \times (RT/zF)$ in the following equation (2) (Nernst equation).

$$E = E0 + 2.303 \times (RT/zF) \times Log(f \times C) \quad \text{Equation (2)}$$

E: potential of selective electrode
E0: constant potential determined by measurement system
z: valence of ions to be measured
F: Faraday constant
R: gas constant
T: absolute temperature
f: activity coefficient
C: ion concentration

A concentration $C_{is}$ of the internal standard solution is obtained according to the following equations (3) and (4) based on the measured electromotive force $EMF_{IS}$ of the internal standard solution.

$$C_{is} = C_L \times 10^a \quad \text{Equation (3)}$$

$$a = (EMF_{IS} - EMF_L)/SL \quad \text{Equation (4)}$$

Although a specific calibration procedure is described above, the calibration procedure is not limited thereto. In the above example, after the measurement of the standard solution having the known low concentration, the measurement of the standard solution having the known high concentration is executed. Alternatively, the order may be reversed.

(Measurement of Specimen Liquid)

Measurement of a specimen liquid is also executed in the same procedure as described above. After the above-described calibration is executed, the measurement of the specimen liquid is executed. In the measurement of the specimen liquid, the measurement of the specimen liquid and the measurement of the internal standard solution are executed alternately and continuously.

After the calibration is executed, an analysis is executed using serum, urine, or the like as a specimen. Specifically, the specimen liquid is aliquoted into the diluent tank 104 using a specimen aliquoting nozzle (not shown), then the diluent in the diluent bottle 105 is aliquoted into the diluent tank 104 using the diluent syringe 106, and the specimen is diluted with a set ratio to generate a diluted specimen liquid. Next, the sipper syringe 108 is operated to introduce the diluted specimen liquid in the diluent tank 104 into the flow paths of the ion selective electrodes 111 to 113. Further, the reference electrolyte is introduced from the reference electrolyte bottle 114 into the flow path of the reference electrode 116. Accordingly, the reference electrolyte and the diluted specimen liquid come into contact with each other. Thereafter, the control unit 120 measures a potential difference (an electromotive force $EMF_s$ of the diluted specimen liquid) between the reference electrode 116 and each of the ion selective electrodes 111 to 113.

When the electromotive force of the diluted specimen liquid is measured, the diluted specimen liquid remained in the diluent tank 104 is discarded to a waste liquid tank. Thereafter, the internal standard solution in the internal standard solution bottle 117 is newly aliquoted into the diluent tank 104 using the internal standard solution syringe 118. Next, the sipper syringe 108 is operated to introduce the internal standard solution in the diluent tank 104 into the flow paths of the ion selective electrodes 111 to 113. Further, the reference electrolyte is introduced from the reference electrolyte bottle 114 into the flow path of the reference electrode 116. Accordingly, the reference electrolyte and the internal standard solution come into contact with each other, and the control unit 120 measures a potential difference (an electromotive force $EMF_{IS}$ of the internal standard solution) between the reference electrode 116 and each of the ion selective electrodes 111 to 113.

A concentration $C_s$ of the specimen is calculated using the following equations (5) and (6) based on the slope sensitivity SL and the concentration $C_{is}$ of the internal standard solution.

$$C_s = C_{is} \times 10^b \quad \text{Equation (5)}$$

$$b = (EMF_{IS} - EMF_S)/SL \quad \text{Equation (6)}$$

The above calculation equations are basic equations. In the device according to the present embodiment, an internal standard solution having a constant concentration is also measured before and after the measurement of the diluted specimen liquid. Measured values of the diluted specimen liquid are corrected based on measured values of the internal standard solution. Therefore, even when a gentle potential fluctuation (a potential drift phenomenon) caused by a surface change of a sensitive membrane of each of the ion selective electrodes 111 to 113 or a temperature change, accurate measurement can be achieved.

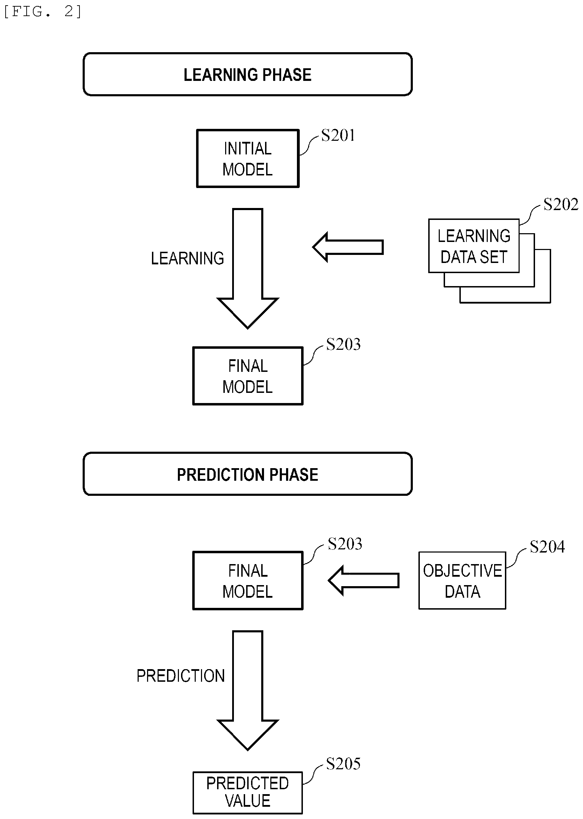

FIG. 2 is a schematic diagram showing a step (a learning phase) of constructing a final model and a step (a prediction phase) of predicting an unknown result using the constructed final model according to Embodiment 1.

(Learning Phase)

In the learning phase, machine learning of an initial model is performed using a plurality of learning data sets selected from a library in which data is collected, and a final model (a trained model) is constructed.

First, the initial model is set (step S201). Then, training of the initial model is performed using a plurality of learning data sets selected from the library (step S202), and the final model (hereinafter, the final model is appropriately referred to as a trained model) is constructed (step S203) . An input parameter is set in the initial model. Setting of the parameter affects properties of the model. In the learning process, a calculation is executed such that an internal parameter such as a weighting factor in the model is gradually adjusted to reduce a risk that the trained model outputs an incorrect answer when a measured value of the electrolyte concentration measuring device 100 is input.

(Prediction Phase)

When the final model is predicted using objective data (step S204) other than the learning data, and a good predicted value can be obtained, it can be said that the model functions well (step S205) .

In the learning according to Embodiment 1, a supervised learning data set is used. This is a method in which a data set obtained by setting an input item and a ground truth value of the input item as learning data is given to a model and the model learns a rule that leads to ground truth.

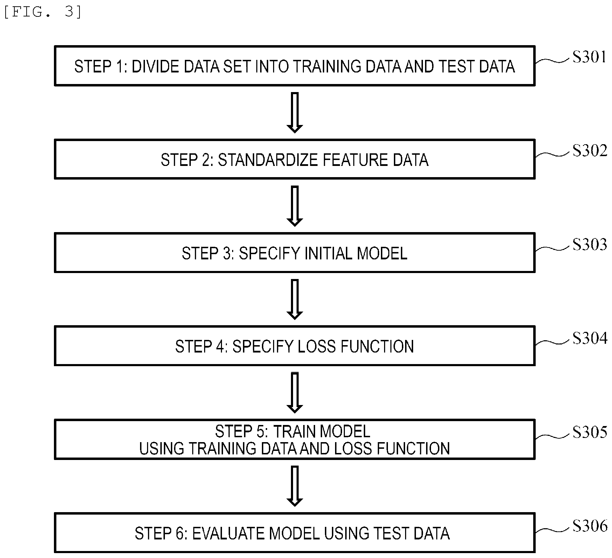

FIG. 3 is a flowchart showing a flow of training the initial model.

Step 1: All learning data sets are divided into training data and test data. The training data is used in step 5, the test data is used in step 6, and a cross-validation method is applied in which different kinds of data are used for training and evaluation of a prediction model. A division ratio between the training data and the test data is 7:3 or 8:2 (step S301).

Step 2: Standardization is executed in which when different kinds of feature data are mixed, the feature data is standardized and reference is set such that the feature data has the same scale. An average value of the feature data is zero and a standard deviation is 1 by executing the standardization (step S302).

Step 3: The initial model is specified. In the initial model, an equation of the initial model is prepared for each algorithm, and a precondition for the model is determined. An output of the initial model is a calculation result of an input and a parameter (step S303).

Step 4: A loss function is specified. The loss function is used to train the model of a stage using a function for evaluating an error. A definition of the error and an equation of the loss function are different for each algorithm, and the loss function is a function that determines a premise of an algorithm in the initial model and a set (step S304).

Step 5: In the training of the model, an error of the training data is calculated using the initial model and the loss function, and a parameter for minimizing the loss function is calculated. The loss function compares an output of the initial model with a ground truth value to calculate an error. The parameter is updated to reduce the error (step S305).

Step 6: The model is evaluated by outputting a predicted value of the test data using the parameter obtained in step 5 and the test data and comparing the predicted value with the ground truth value (step S306).

(Method for Obtaining Selectivity Coefficient of General Interfering Ions)

Here, an example of a method for obtaining a selectivity coefficient of general interfering ions in an ion selective electrode will be described. An objective ion concentration ($C_i$) obtained by measuring a solution (a second solution) containing only objective ions is subtracted from an objective ion concentration ($C_j$) obtained by measuring a solution (a first solution) containing objective ions and interfering ions for which a selectivity coefficient is desired to be obtained, and the selectivity coefficient is obtained by dividing the subtraction result by a known objective ion concentration (C) (see the following equation (7)). The objective ion concentration of the first solution and the objective ion concentration of the second solution are set to the same ion concentration (C). Since a solution containing the subjective ions and the interfering ions is used, the method is called a mixed solution method.

Selectivity coefficient of interfering ions = ($C_j$ - $C_i$) /C          Equation (7)

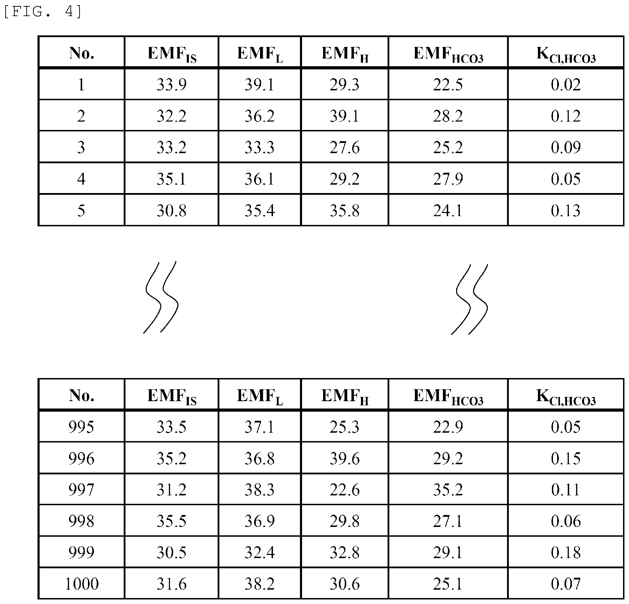

FIG. 4 is a diagram showing a plurality of learning data sets. FIG. 4 shows a part of 1000 learning data sets. The learning data sets shown in FIG. 4 include measured potentials ($EMF_{IS}$, $EMF_L$, and $EMF_H$) of three types of solutions having the same bicarbonate ion concentration and different chloride ion concentrations as feature data, and include, as a ground truth value for a combination of the feature data, a selectivity coefficient ($K_{Cl,HCO3}$) of the bicarbonate ions or a value (hereinafter referred to as a predicted potential as appropriate) ($EMF_{HCO3}$) corresponding to a concentration of the bicarbonate ions. The value ($EMF_{HCO3}$) corresponding to the concentration of the bicarbonate ions is a measured potential of a solution having a bicarbonate ion concentration different from that of the three types of solutions measured to obtain the selectivity coefficient, and the selectivity coefficient ($K_{Cl,HCO3}$) of the bicarbonate ions is a value calculated based on the measured potential. When the measured potentials ($EMF_{IS}$, $EMF_L$, and $EMF_H$) of three types of solutions having the same bicarbonate ion concentration and different chloride ion concentrations measured by the electrolyte concentration measuring device 100 are input to the final model optimized using the plurality of learning data sets shown in FIG. 4, a predicted potential ($EMF_{HCO3}$) for obtaining the selectivity coefficient or a predicted selectivity coefficient ($K_{Cl,HCO3}$) of the bicarbonate ions is output.

When the predicted potential ($EMF_{HCO3}$) for obtaining the selectivity coefficient is selected as an output, it is possible to obtain the selectivity coefficient ($K_{Cl,HCO3}$) of the bicarbonate ions by using the potentials ($EMF_{IS}$, $EMF_L$, and $EMF_H$) measured by the electrolyte concentration measuring device 100 and the predicted potential ($EMF_{HCO3}$).

As described above, the electrolyte concentration measuring device 100 according to Embodiment 1 measures three types of solutions having the same interfering ion concentration and different objective ion concentrations in the calibration. In Embodiment 1, the measured potentials of the three types of solutions measured in the calibration are input to the final model, and the selectivity coefficient ($K_{Cl,HCO3}$) of the bicarbonate ions or the value ($EMF_{HCO3}$) corresponding to the concentration of the bicarbonate ions is output.

In the related art, it is difficult to obtain a selectivity coefficient after shipment of an anion selective electrode. In the present embodiment, the selectivity coefficient of interfering ions can be obtained in real time, such as at a timing of calibration. Therefore, a state of the anion selective electrode can be grasped, and reliability of an assumed value can be improved.

(Correlation between Input and Output of Final Model)

Learning results obtained by various algorithms using the plurality of learning data sets shown in FIG. 4 are shown as below. As a coefficient of determination indicating a correlation between the test data and a predicted value is closer to 1, it indicates an algorithm having better performance (the correlation is higher).

(Model 1)

Algorithm: multiple regression

Number of data sets: 1000

Division ratio between training data and test data: 8:2

Feature data: $EMF_{IS}$, $EMF_L$, $EMF_H$

Ground truth value: $EMF_{HCO3}$ or $K_{Cl,HCO3}$

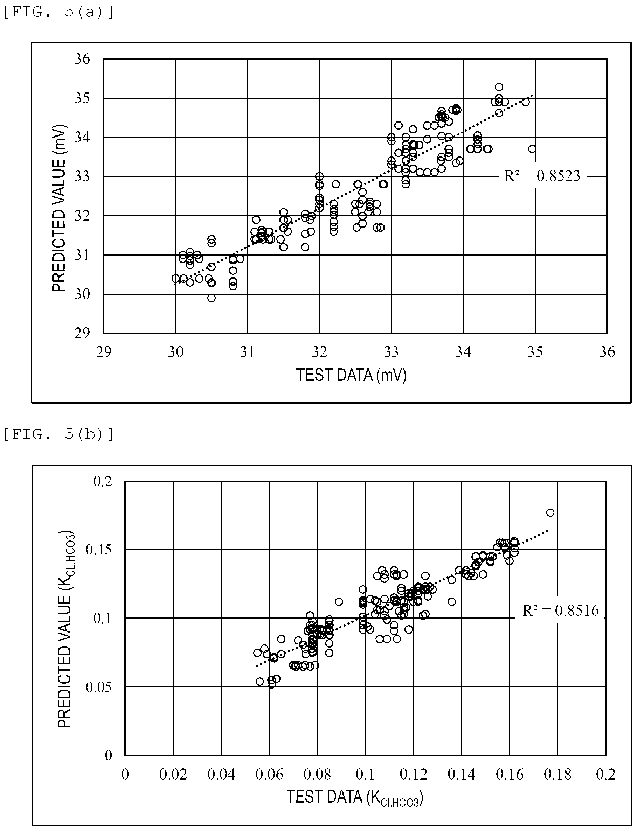

FIG. 5(a) and FIG. 5(b) show plots of test data and predicted values. A mean squared error and a coefficient of determination are shown in Table 1.

[Table 1]

| Ground truth value | $EMF_{HCO3}$ | | $K_{Cl,HCO3}$ | |
|---|---|---|---|---|
| | Training Data | Test Data | Training Data | Test Data |
| Mean squared error | 0.23 | 0.33 | 0.00012 | 0.00035 |
| Coefficient of determination | 0.852 | | 0.852 | |

(Model 2)

    Algorithm: stochastic gradient descent regression
Parameter: learning rate 0.01
Number of data sets: 1000
Division ratio between training data and test data: 8:2
Feature data: $EMF_{IS}$, $EMF_{L}$, $EMF_{H}$
Ground truth value: $EMF_{HCO3}$ or $K_{Cl,HCO3}$

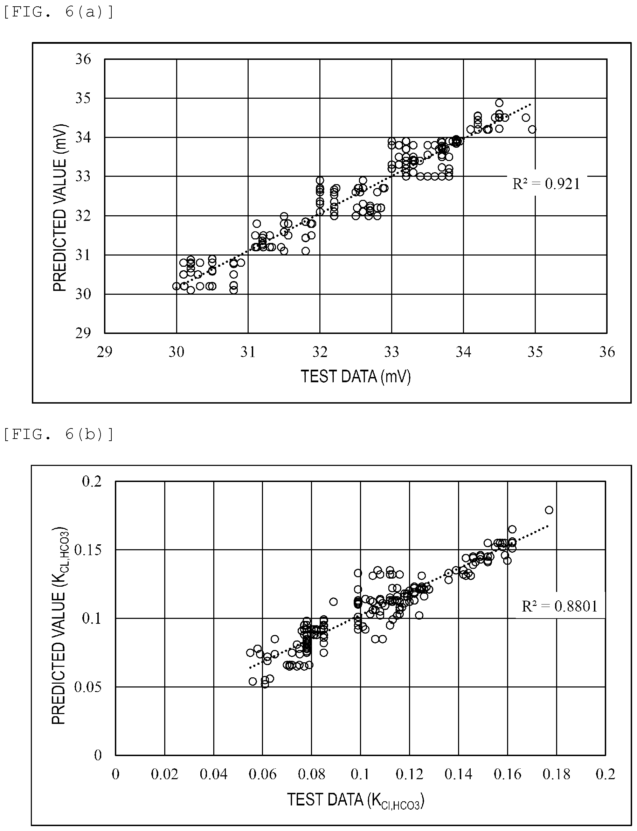

FIG. 6(a) and FIG. 6(b) show plots of test data and predicted values. A mean squared error and a coefficient of determination are shown in Table 2.

[Table 2]

| Ground truth value | $EMF_{HCO3}$ | | $K_{Cl,HCO3}$ | |
|---|---|---|---|---|
| | Training Data | Test Data | Training Data | Test Data |
| Mean squared error | 0.23 | 0.36 | 0.00022 | 0.00023 |
| Coefficient of determination | 0.921 | | 0.880 | |

(Model 3)

    Algorithm: polynomial regression
Parameter: cubic polynomial
Number of data sets: 1000
Division ratio between training data and test data: 8:2
Feature data: $EMF_{IS}$, $EMF_{L}$, $EMF_{H}$
Ground truth value: $EMF_{HCO3}$ or $K_{Cl,HCO3}$

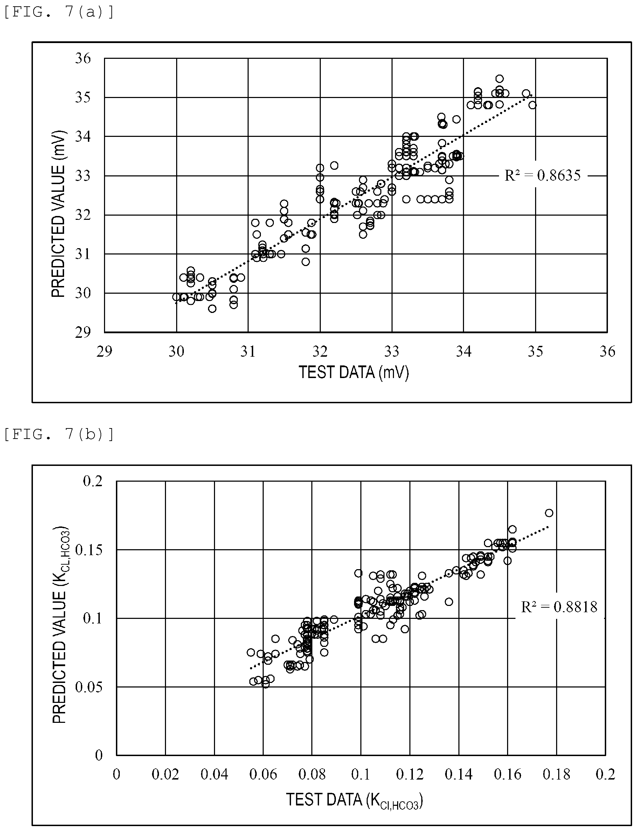

FIG. 7(a) and FIG. 7(b) show plots of test data and predicted values. A mean squared error and a coefficient of determination are shown in Table 3.

[Table 3]

| Ground truth value | $EMF_{HCO3}$ | | $K_{Cl,HCO3}$ | |
|---|---|---|---|---|
| | Training Data | Test Data | Training Data | Test Data |
| Mean squared error | 0.20 | 0.17 | 0.00043 | 0.00033 |
| Coefficient of determination | 0.863 | | 0.881 | |

(Model 4)

    Algorithm: Ridge regression

Parameter: cubic polynomial, L2 regularization $\alpha = 0.0001$

Number of data sets: 1000

Division ratio between training data and test data: 8:2

Feature data: $EMF_{IS}$, $EMF_L$, $EMF_H$

Ground truth value: $EMF_{HCO3}$ or $K_{Cl,HCO3}$

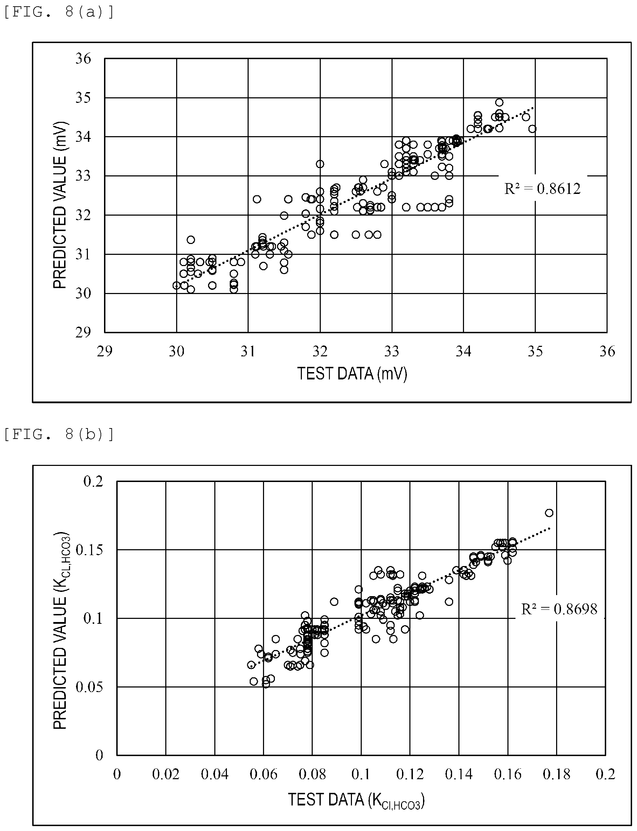

FIG. 8(a) and FIG. 8(b) show plots of test data and predicted values. A mean squared error and a coefficient of determination are shown in Table 4.

[Table 4]

| Ground truth value | $EMF_{HCO3}$ | | $K_{Cl,HCO3}$ | |
|---|---|---|---|---|
| | Training Data | Test Data | Training Data | Test Data |
| Mean squared error | 0.19 | 0.23 | 0.00011 | 0.00043 |
| Coefficient of determination | 0.861 | | 0.869 | |

(Model 5)

Algorithm: Lasso regression

Parameter: cubic polynomial, L1 regularization $\alpha = 0.01$

Number of data sets: 1000

Division ratio between training data and test data: 8:2

Feature data: $EMF_{IS}$, $EMF_L$, $EMF_H$

Ground truth value: $EMF_{HCO3}$ or $K_{Cl,HCO3}$

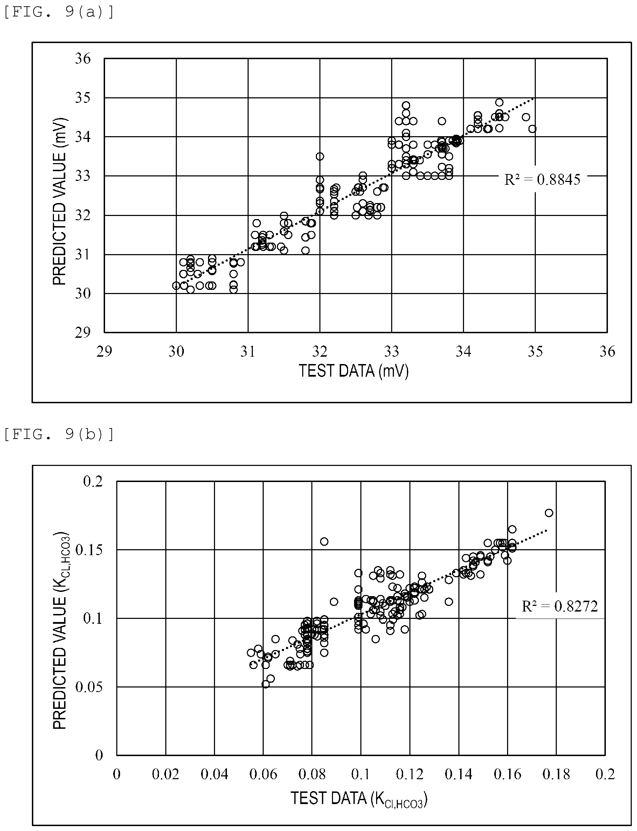

FIG. 9(a) and FIG. 9(b) show plots of test data and predicted values. A mean squared error and a coefficient of determination are shown in Table 5.

[Table 5]

| Ground truth value | $EMF_{HCO3}$ | | $K_{Cl,HCO3}$ | |
|---|---|---|---|---|
| | Training Data | Test Data | Training Data | Test Data |
| Mean squared error | 0.21 | 0.34 | 0.00033 | 0.00043 |
| Coefficient of determination | 0.884 | | 0.827 | |

(Model 6)

Algorithm: Elastic Net regression

Parameter: cubic polynomial, L1 + L2 regularization $\alpha = 0.001$, l1_ratio = 0.3

Number of data sets: 1000

Division ratio between training data and test data: 8:2

Feature data: $EMF_{IS}$, $EMF_L$, $EMF_H$

Ground truth value: $EMF_{HCO3}$ or $K_{Cl,HCO3}$

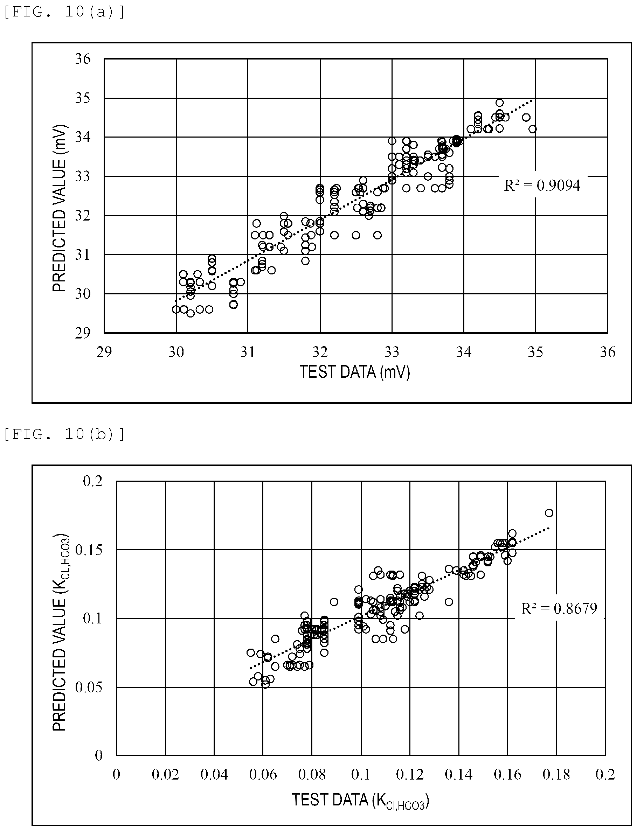

FIG. 10(a) and FIG. 10(b) show plots of test data and predicted values. A mean squared error and a coefficient of determination are shown in Table 6.

[Table 6]

| Ground truth value | $EMF_{HCO3}$ | | $K_{Cl,HCO3}$ | |
|---|---|---|---|---|
| | Training Data | Test Data | Training Data | Test Data |
| Mean squared error | 0.21 | 0.30 | 0.00021 | 0.00016 |
| Coefficient of determination | 0.909 | | 0.868 | |

(Model 7)

Algorithm: Support vector regression

Parameter: linear kernel, C = 1.0, $\varepsilon$ = 0.3

Number of data sets: 1000

Division ratio between training data and test data: 8:2

Feature data: $EMF_{IS}$, $EMF_L$, $EMF_H$

Ground truth value: $EMF_{HCO3}$ or $K_{Cl,HCO3}$

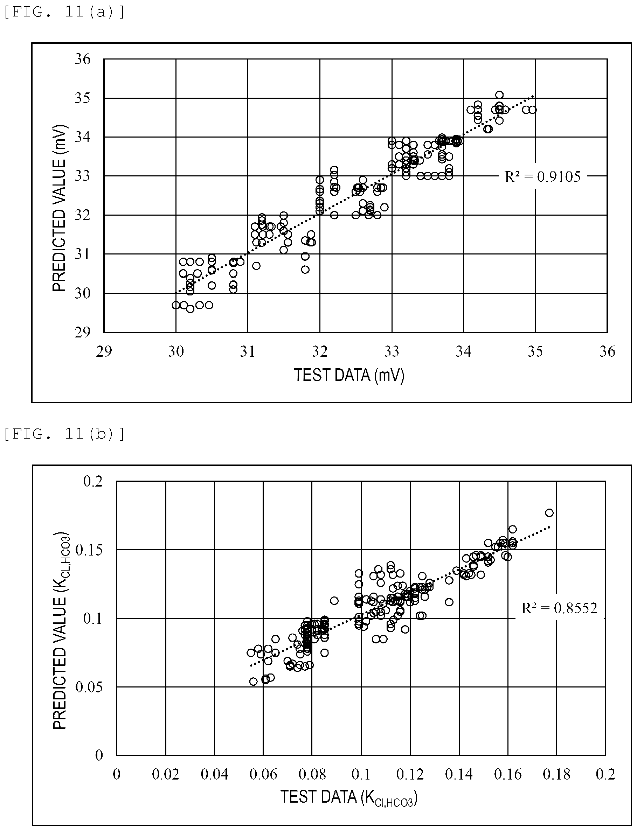

FIG. 11(a) and FIG. 11(b) show plots of test data and predicted values. A mean squared error and a coefficient of determination are shown in Table 7.

[Table 7]

| Ground truth value | $EMF_{HCO3}$ | | $K_{Cl,HCO3}$ | |
|---|---|---|---|---|
| | Training Data | Test Data | Training Data | Test Data |
| Mean squared error | 0.20 | 0.38 | 0.00036 | 0.00044 |
| Coefficient of determination | 0.910 | | 0.855 | |

(Model 8)

Algorithm: Support vector regression with Gaussian kernel

Parameter: Gaussian kernel, C = 1.0, $\varepsilon$ = 0.3, $\gamma$ = 001

Number of data sets: 1000

Division ratio between training data and test data: 8:2

Feature data: $EMF_{IS}$, $EMF_L$, $EMF_H$

Ground truth value: $EMF_{HCO3}$ or $K_{Cl,HCO3}$

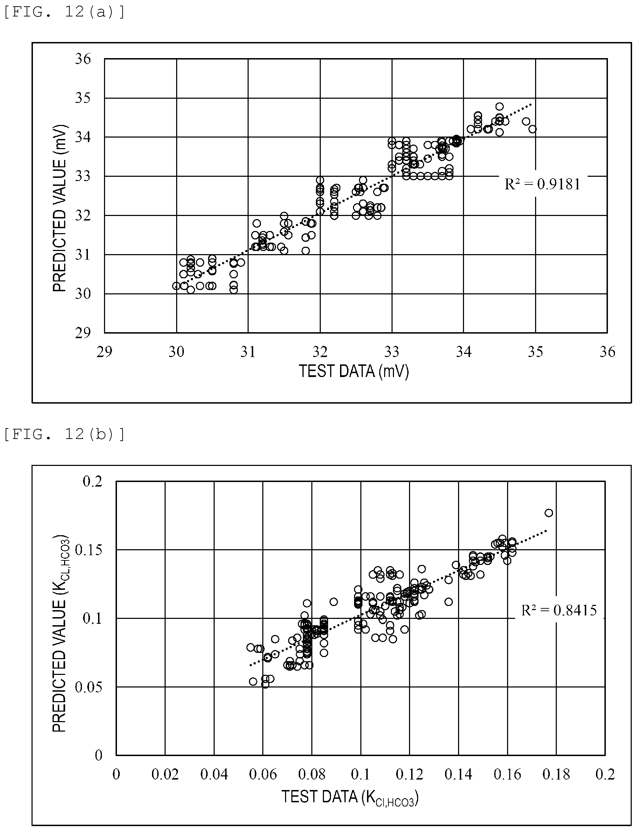

FIG. 12(a) and FIG. 12(b) show plots of test data and predicted values. A mean squared error and a coefficient of determination are shown in Table 8.

[Table 8]

| Ground truth value | $EMF_{HCO3}$ | | $K_{Cl,HCO3}$ | |
|---|---|---|---|---|
| | Training Data | Test Data | Training Data | Test Data |
| Mean squared error | 0.20 | 0.38 | 0.00023 | 0.00033 |
| Coefficient of determination | 0.918 | | 0.841 | |

(Model 9)

Algorithm: decision tree
Parameter: max_depth = 8
Number of data sets: 1000
Division ratio between training data and test data: 8:2
Feature data: $EMF_{IS}$, $EMF_L$, $EMF_H$
Ground truth value: $EMF_{HCO3}$ or $K_{Cl,HCO3}$

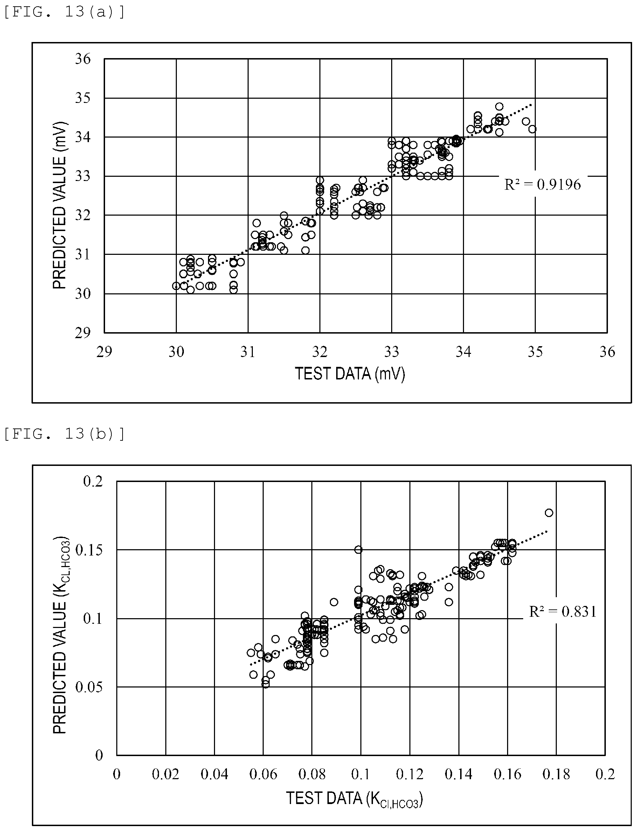

FIG. 13(a) and FIG. 13(b) show plots of test data and predicted values. A mean squared error and a coefficient of determination are shown in Table 9.

[Table 9]

| Ground truth value | $EMF_{HCO3}$ | | $K_{Cl,HCO3}$ | |
|---|---|---|---|---|
| | Training Data | Test Data | Training Data | Test Data |
| Mean squared error | 0.26 | 0.11 | 0.00017 | 0.00024 |
| Coefficient of determination | 0.919 | | 0.831 | |

(Model 10)

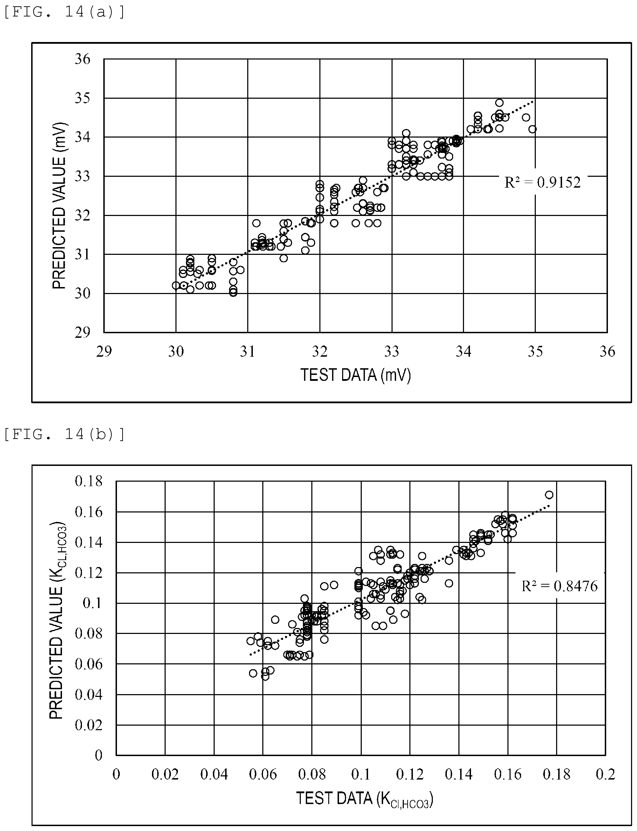

Algorithm: random forest
Parameter: bootstrap, n_estimators = 1000, max_depth = None
Number of data sets: 1000
Division ratio between training data and test data: 8:2
Feature data: $EMF_{IS}$, $EMF_L$, $EMF_H$
Ground truth value: $EMF_{HCO3}$ or $K_{Cl, HCO3}$
FIG. 14(a) and FIG. 14(b) show plots of test data and predicted values. A mean squared error and a coefficient of determination are shown in Table 10.

[Table 10]

| Ground truth value | $EMF_{HCO3}$ | | $K_{Cl,HCO3}$ | |
|---|---|---|---|---|
| | Training Data | Test Data | Training Data | Test Data |
| Mean squared error | 0.22 | 0.05 | 0.00064 | 0.00055 |
| Coefficient of determination | 0.915 | | 0.847 | |

(Model 11)

Algorithm: XGBoost

Parameter: learning_rate = 1

Number of data sets: 1000

Division ratio between training data and test data: 8:2

Feature data: $EMF_{IS}$, $EMF_L$, $EMF_H$

Ground truth value: $EMF_{HCO3}$ or $K_{Cl,HCO3}$

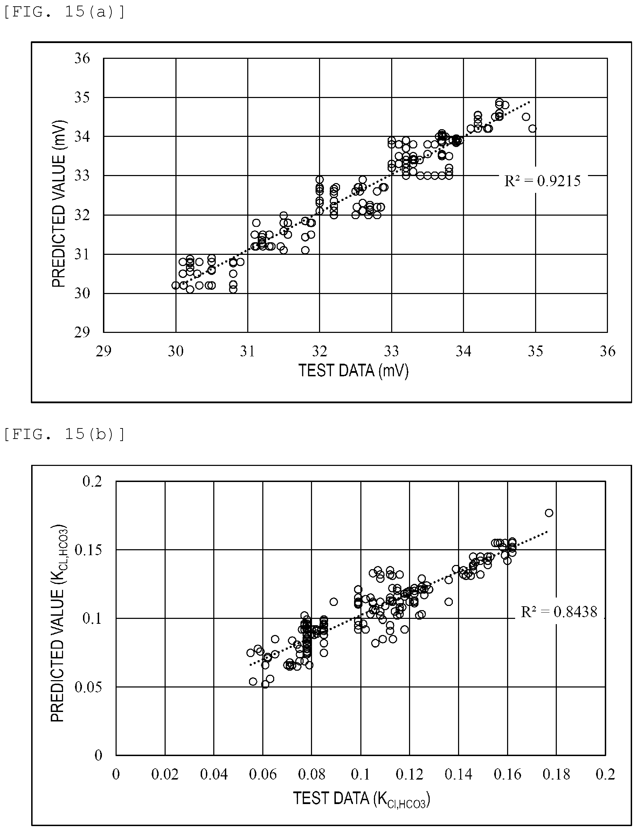

FIG. 15(a) and FIG. 15(b) show plots of test data and predicted values. A mean squared error and a coefficient of determination are shown in Table 11.

[Table 11]

| Ground truth value | $EMF_{HCO3}$ | | $K_{Cl,HCO3}$ | |
|---|---|---|---|---|
| | Training Data | Test Data | Training Data | Test Data |
| Mean squared error | 0.32 | 0.05 | 0.00055 | 0.00061 |
| Coefficient of determination | 0.921 | | 0.0844 | |

(Model 12)

Algorithm: multilayer perceptron

Parameter: hidden layer 2, activation function = relu, output layer 1, optimizer = sgd

Number of data sets: 1000

Division ratio between training data and test data: 8:2

Feature data: $EMF_{IS}$, $EMF_L$, $EMF_H$

Ground truth value: $EMF_{HCO3}$ or $K_{Cl,HCO3}$

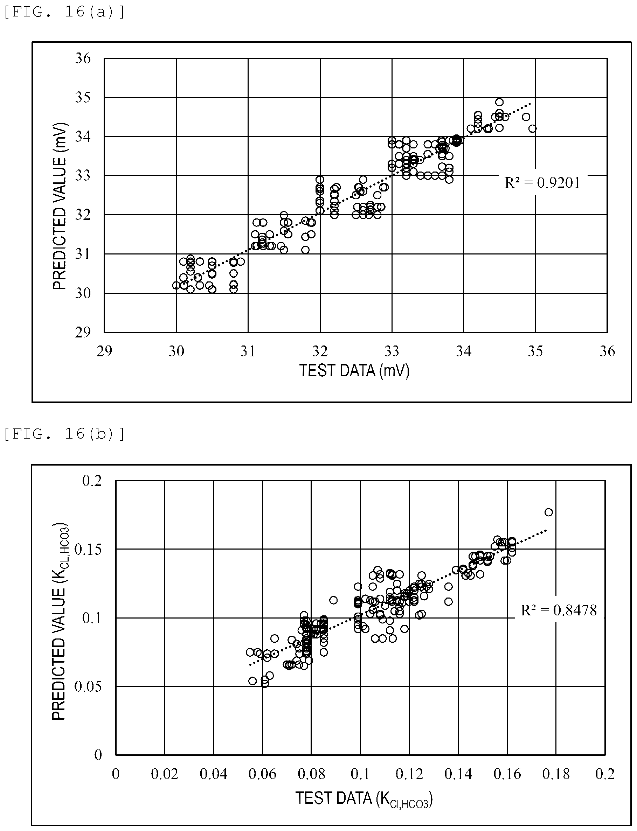

FIG. 16(a) and FIG. 16(b) show plots of test data and predicted values. A mean squared error and a coefficient of determination are shown in Table 12.

[Table 12]

| Ground truth value | $EMF_{HCO3}$ | | $K_{Cl,HCO3}$ | |
|---|---|---|---|---|
| | Training Data | Test Data | Training Data | Test Data |
| Mean squared error | 0.25 | 0.15 | 0.00042 | 0.00053 |
| Coefficient of determination | 0.920 | | 0.848 | |

As a result of machine learning using the above-described various algorithms, a high result in which the coefficient of determination is 0.831 or more was obtained. From the above results, it was found that there was a correlation between measured potentials of a plurality of (three in the present embodiment) solutions having the same bicarbonate ion concentration and the selectivity coefficient of the bicarbonate ions or a value corresponding to the concentration of the bicarbonate ions. It was found that the selectivity coefficient of the bicarbonate ions can be estimated with high accuracy based on the measured potentials of the plurality of solutions having the same bicarbonate ion concentration by machine learning using any algorithm.

In the present embodiment, the storage unit 121 records a function (a trained model) obtained from the stochastic gradient descent regression which is an algorithm having a coefficient of determination closest to 1.

The obtained mean squared error and coefficient of determination differ depending on a combination of learning data sets used for learning and a batch size. Further, the obtained mean squared error and coefficient of determination differ depending on a combination of parameters set in algorithms. Accordingly, setting conditions such as algorithms of machine learning are not limited to those described above.

(1) Deterioration Determination Processing

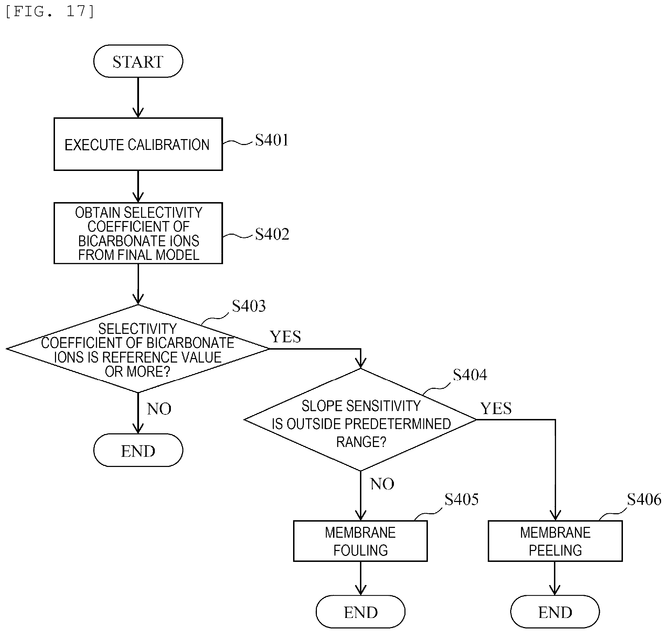

FIG. 17 is a flowchart showing deterioration determination processing according to Embodiment 1. The deterioration determination processing determines a deterioration state of the anion selective electrode based on a prediction result obtained using machine learning. The deterioration determination processing will be described with reference to FIG. 17. A deterioration determination program for causing the computer system 200 to execute the deterioration determination processing shown in FIG. 17 is stored in the storage unit 121. The processor 201 reads and executes the deterioration determination program stored in the storage unit 121.

The following items are assumed as factors causing deterioration. It is possible to specify an assumed deterioration factor by comparing the selectivity coefficient of the bicarbonate ions output from the final model with a reference value according to the flowchart shown in FIG. 17.

  1. Membrane fouling
  2. Membrane peeling

In general, performance of the anion selective electrode is lowered as time elapses from a manufacturing date. In addition, the performance of the electrode is lowered as a frequency of use in measurement of a specimen liquid or the like increases. The electrolyte concentration measuring device 100 incorporated with the anion selective electrode is provided with a mechanism of generating an alarm in response to a fluctuation in a measured potential or a slope sensitivity, but is not provided with an alarm generation function related to a fluctuation in a selectivity coefficient of interfering ions. Therefore, it is difficult to predict a problem caused by a fluctuation in the selectivity coefficient of interfering ions. In Embodiment 1, since the selectivity coefficient of interfering ions can be obtained at the timing of calibration or at any timing, the selectivity coefficient of interfering ions can be obtained in real time. Therefore, it is possible to immediately grasp a problem caused by a fluctuation in the selectivity coefficient of interfering ions, and it is expected to greatly improve reliability of measured data.

In Embodiment 1, when the calibration is executed, the selectivity coefficient of interfering ions of the anion selective electrode is automatically calculated, and the deterioration determination processing is executed using the calculated selectivity coefficient of interfering ions. At time other than when the calibration is executed, the control unit 120 may calculate the selectivity coefficient of interfering ions and execute the deterioration determination processing at a timing when an operator input a start instruction of the deterioration determination processing or at a timing of a preset start time of the deterioration determination processing.

First, the computer system 200 performs control to execute calibration of the anion selective electrode (step S401). Measured results (for example, $EMF_{IS}$, $EMF_L$, and $EMF_H$) of three types of solutions having the same bicarbonate ion concentration and different chloride ion concentrations can be obtained by the calibration. Subsequently, the computer system 200 inputs the measured results of the calibration to the final model and obtains a selectivity coefficient of bicarbonate ions from the final model (Step S402). A value obtained in step S402 may be a value corresponding to a concentration of the bicarbonate ions.

As described above, the selectivity coefficient of the bicarbonate ions can be obtained from the final model. That is, a method for obtaining a selectivity coefficient of interfering ions includes:

  measuring three types of solutions having the same bicarbonate ion concentration and different chloride ion concentrations by calibration to obtain measured results (for example, $EMF_{IS}$, $EMF_L$, and $EMF_H$);
  inputting the measured results (for example, $EMF_{IS}$, $EMF_L$, and $EMF_H$) to the final model; and
  obtaining the selectivity coefficient of the interfering ions or a value corresponding to a concentration of the interfering ions from the final model.

Next, the computer system 200 determines whether the obtained selectivity coefficient of the bicarbonate ions is a reference value or more (step S403). When the selectivity coefficient of the bicarbonate ions is less than the reference value (step S403: No), the flowchart ends. When the selectivity coefficient of the bicarbonate ions is the reference value or more (step S403: Yes), the computer system 200 determines whether the slope sensitivity is outside a predetermined range (step S404). When it is determined that the slope sensitivity is not outside the predetermined range (step S404: No), the computer system 200 issues a membrane fouling alarm (step S405), and when it is determined that the slope sensitivity is outside the predetermined range (step S404: Yes), the computer system 200 issues a membrane peeling alarm (step S406).

Even when there is no change in the measured potential or the slope sensitivity of the anion selective electrode, the selectivity coefficient of the bicarbonate ions may fluctuate. When the selectivity of the bicarbonate ions fluctuates, measured values of chloride ions do not indicate accurate values unless calibration is executed. In Embodiment 1, the selectivity coefficient of the bicarbonate ions can be predicted. Even when there is no change in the slope sensitivity, a membrane fouling alarm is issued when the selectivity coefficient of the bicarbonate ions fluctuates. Since the selectivity coefficient of the bicarbonate ions is improved by performing membrane cleaning, it is possible to immediately grasp a problem caused by a fluctuation in the selectivity coefficient of the bicarbonate ions, and it is expected to greatly improve reliability of measured data.

(2) Maintenance Support Processing

In the deterioration determination processing shown in FIG. 17, a process procedure when any one of the deterioration factors is issued will be described.

(2-1) Maintenance Support Processing corresponding to Membrane Fouling Alarm

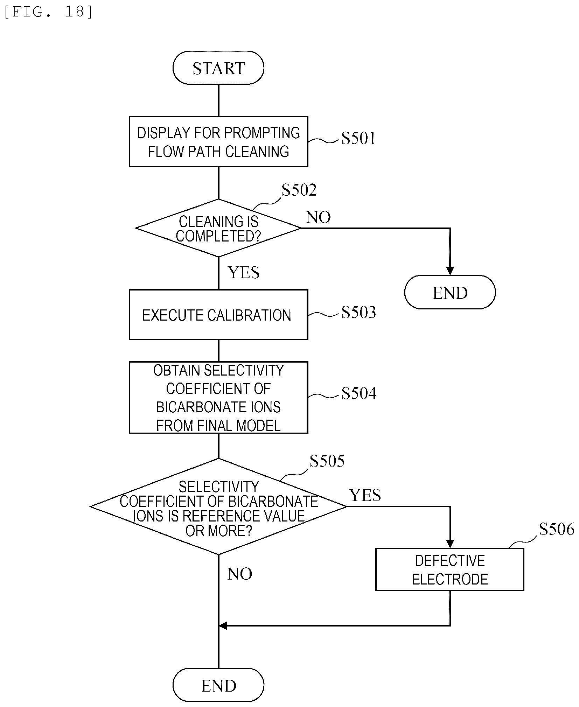

FIG. 18 is a processing flow showing maintenance support processing corresponding to a membrane fouling alarm. When the computer system 200 issues the membrane fouling alarm, the computer system 200 performs display for prompting an operator to perform flow path cleaning on the display unit 122 (step S501). Subsequently, the computer system 200 determines whether the cleaning is completed (step S502). Specifically, the computer system 200 displays a screen for confirming whether the cleaning is completed on the display unit 122. When information indicating that the cleaning is completed is input by the operator via the input unit 123 such as a keyboard, it is determined that the cleaning is completed, otherwise, it is determined that the cleaning is not completed.

When it is determined that the cleaning is completed (step S502: Yes), the computer system 200 performs control to execute calibration (step S503). Measured results (for example, $EMF_{IS}$, $EMF_L$, and $EMF_H$) of three types of solutions having the same bicarbonate ion concentration and different chloride ion concentrations can be obtained by the calibration. Subsequently, the computer system 200 inputs the measured results of the calibration to the final model and obtains a selectivity coefficient of bicarbonate ions from the final model (Step S504).

Next, the computer system 200 determines whether the obtained selectivity coefficient of the bicarbonate ions is a reference value or more (step S505). When the selectivity coefficient of the bicarbonate ions is the reference value or more (step S505: Yes), the computer system 200 issues a defective electrode alarm (step S506) and ends the processing. On the other hand, when the selectivity coefficient of the bicarbonate ions is less than the reference value (step S505: No), it is determined that the membrane fouling is eliminated by the cleaning, information indicating no abnormality is issued, and the present flowchart ends.

(2-2) Maintenance Support Processing corresponding to Membrane Peeling Alarm

As described above, the electrolyte concentration measuring device 100 incorporated with the anion selective electrode is provided with a mechanism of generating an alarm in response to a fluctuation in the measured potential or the slope sensitivity. When membrane peeling occurs, there is a high possibility that an alarm is issued, but there may be a case in which no alarm is issued when a fluctuation in the slope sensitivity is small. In Embodiment 1, according to prediction of the selectivity coefficient of interfering ions, it is possible to know a problem at a timing earlier than a timing when an alarm of the measured potential or the slope sensitivity is issued, and it can be expected to greatly improve reliability. When a membrane peeling alarm is issued, the computer system 200 issues a defective electrode alarm and ends the processing.

Next, a comparison result between the reference value and an output (an electromotive force of the bicarbonate ions, or the selectivity coefficient of the bicarbonate ions) obtained by inputting the measured values ($EMF_{IS}$, $EMF_L$, and $EMF_H$) into the final model will be described, the measured values ($EMF_{IS}$, $EMF_L$, and $EMF_H$) being obtained by the calibration of the electrolyte concentration measuring device according to Embodiment 1. Here, four examples of cases A to D will be described.

(Case A)

In Case A, a predicted value $EMF_{HCO3}$ was obtained by inputting measured values ($EMF_{IS}$, $EMF_L$, and $EMF_H$) in the following table 13 obtained by the calibration into a function (a trained model) of an algorithm recorded in the storage unit 121. Next, the selectivity coefficient $K_{Cl,HCO3}$ of the bicarbonate ions was obtained according to the following equation (8), and was compared with the reference value.

$$K_{Cl,HCO3} = 10^{(EMFHCO3-EMFIS)/SL-1} \qquad \text{Equation (8)}$$

[Table 13]

| Electrode Number | $EMF_{IS}$ (mV) | $EMF_L$ (mV) | $EMF_H$ (mV) | Slope sensitivity (mV/dec.) |
|---|---|---|---|---|
| 1 | 33.5 | 38.2 | 28.6 | -52.2 |

As a result, the selectivity coefficient of the bicarbonate ions was 0.35, which was larger than the reference value of 0.1 by 0.25. Since there was no decrease in the slope sensitivity SL, a membrane fouling alarm was issued according to the flowchart shown in FIG. 17. A screen for prompting an operator to perform flow path cleaning was displayed on the display unit 122.

(Case B)

In Case B, the selectivity coefficient $K_{Cl,HCO3}$ of the bicarbonate ions was obtained by inputting measured values ($EMF_{IS}$, $EMF_L$, and $EMF_H$) in the following Table 14 obtained by the calibration into a function of an algorithm recorded in the storage unit 121, and was compared with the reference value.

[Table 14]

| Electrode Number | $EMF_{IS}$ (mV) | $EMF_L$ (mV) | $EMF_H$ (mV) | Slope sensitivity (mV/dec.) |
|---|---|---|---|---|
| 2 | 31.5 | 36.2 | 28.9 | -50.2 |

As a result, the selectivity coefficient of the bicarbonate ions was 0.30, which was larger than the reference value of 0.1 by 0.20. Since there was no decrease in the slope sensitivity SL, a membrane fouling alarm was issued according to the flowchart shown in FIG. 17. A screen for prompting an operator to perform flow path cleaning was displayed on the display unit 122.

(Case C)

In Case C, a predicted value $EMF_{HCO3}$ was obtained by inputting measured values ($EMF_{IS}$, $EMF_L$, and $EMF_H$) in the following table 15 obtained by the calibration into a function of an algorithm recorded in the storage unit 121. Next, the selectivity coefficient $K_{Cl,HCO3}$ of the bicarbonate ions was obtained according to the equation (8), and was compared with the reference value.

[Table 15]

| Electrode Number | $EMF_{IS}$ (mV) | $EMF_L$ (mV) | $EMF_H$ (mV) | Slope sensitivity (mV/dec.) |
|---|---|---|---|---|
| 3 | 30.5 | 35.1 | 29.3 | -41.0 |

As a result, the selectivity coefficient of the bicarbonate ions was 0.41, which was larger than the reference value of 0.1 by 0.31. Since there was a decrease in the slope sensitivity SL, a membrane peeling alarm was issued according to the flowchart shown in FIG. 17. A screen indicating a defective electrode alarm was displayed on the display unit 122.

(Case D)

In Case D, the selectivity coefficient $K_{Cl,HCO3}$ of the bicarbonate ions was obtained by inputting measured values ($EMF_{IS}$, $EMF_L$, and $EMF_H$) in the following Table 16 obtained by the calibration into a function of an algorithm recorded in the storage unit 121, and was compared with the reference value.

[Table 16]

| Electrode Number | EMF$_{IS}$ (mV) | EMF$_L$ (mV) | EMF$_H$ (mV) | Slope sensitivity (mV/dec.) |
|---|---|---|---|---|
| 4 | 32.5 | 33.5 | 30.3 | -43.0 |

As a result, the selectivity coefficient of the bicarbonate ions was 0.45, which was larger than the reference value of 0.1 by 0.35. Since there was a decrease in the slope sensitivity SL, a membrane peeling alarm was issued according to the flowchart shown in FIG. 17. Then, a screen indicating a defective electrode alarm was displayed on the display unit 122.

Although the reference value was set to 0.1 in the above cases A to D, the reference value is not limited to 0.1. The reference value may be freely set by a user.

In the flow shown in FIG. 17, a cause of an abnormality was specified by combining a comparison between the selectivity coefficient of the bicarbonate ions and the reference value (S403 in FIG. 17) and a comparison between the slope sensitivity and the predetermined range (S404 in FIG. 17). In a case of only the comparison between the selectivity coefficient of the bicarbonate ions and the reference value, an abnormality may be detected when the selectivity coefficient of the bicarbonate ions is less than the reference value. That is, whether there is an abnormality may be determined by executing the processing in S401 to S403 in the flowchart shown in FIG. 17. Although it is possible to appropriately deal with an abnormality by specifying a cause of the abnormality, it is possible to perform only the determination of the presence or absence of an abnormality even when a processing capability of the control unit 120 is low.

(Effects of Embodiment 1)

The selectivity coefficient of the bicarbonate ions and the value corresponding to a concentration of the bicarbonate ions can be obtained from the final model by inputting the measured potentials of the three types of solutions containing the chloride ions and the bicarbonate ions into the final model. As described above, in Embodiment 1, it is possible to output the selectivity coefficient of the bicarbonate ions or the value corresponding to a concentration of the bicarbonate ions without the need for a dedicated sample for calculating the selectivity coefficient of interfering ions as in PTL 1.

By training the initial model with the supervised learning data set, it is possible to obtain the selectivity coefficient of the bicarbonate ions or the value corresponding to a concentration of the bicarbonate ions from the final model by inputting the measured potentials of the three types of solutions containing the chloride ions and the bicarbonate ions into the final model.

Since the electrolyte concentration measuring device 100 is provided with the storage unit 121 that stores the trained model, it is possible to obtain the selectivity coefficient of the bicarbonate ions or the value corresponding to a concentration of the bicarbonate ions using a local device.

<Embodiment 2>

An electrolyte concentration measuring device periodically measures a control sample for accuracy management. At this time, in a case where a concentration of bicarbonate ions contained in the control sample is different from concentrations of bicarbonate ions contained in the internal standard solution, and in the high-concentration standard solution and the low-concentration standard solution for calibration, a deviation from a package insert value occurs in a display value at the time of measuring the control sample. When a deviation from the package insert value of the control sample occurs, no decrease in the slope sensitivity of the anion selective electrode is observed. There is a possibility that accuracy management of the anion selective electrode is determined to be inappropriate even in a normal state in which there is no fluctuation in the selectivity coefficient of interfering ions. In Embodiment 2, since the selectivity coefficient of interfering ions can be predicted, even when the concentration of the bicarbonate ions contained in the control sample to be used is different from the concentrations of bicarbonate ions contained in the internal standard solution, and in the high-concentration standard solution and the low-concentration standard solution for calibration, a measured value can be corrected, and thus it can be expected to greatly improve reliability of measured data.

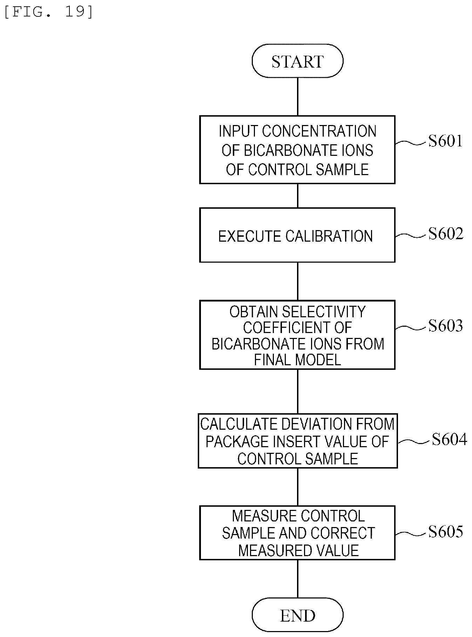

In order to correct the deviation, the computer system 200 executes the following processing according to a flowchart shown in FIG. 19.

An operator inputs a concentration value of the bicarbonate ions contained in the control sample via the input unit 123 or the like (step S601). Then, the computer system 200 executes calibration (step S602), and obtains measured values (EMF$_{IS}$, EMF$_L$, and EMF$_H$). Then, the computer system 200 obtains a predicted value EMF$_{HCO3}$ by inputting the measured values of the calibration shown in Table 17 into a function of an algorithm recorded in the storage unit 121. Next, a selectivity coefficient $K_{Cl,HCO3}$ of the bicarbonate ions was obtained according to the equation (8) (step S603) .

[Table 17]

| Electrode Number | $EMF_{IS}$ (mV) | $EMF_L$ (mV) | $EMF_H$ (mV) | Slope sensitivity (mV/dec.) |
|---|---|---|---|---|
| 3 | 31.5 | 35.8 | 30.4 | -51.0 |

As a result, the selectivity coefficient of the bicarbonate ions was 0.10. Subsequently, the computer system 200 multiplies, by the obtained selectivity coefficient of the bicarbonate ions, a difference between the concentration of the bicarbonate ions contained in the control sample and the concentrations of the bicarbonate ions contained in the internal standard solution and in the high-concentration standard solution and the low-concentration standard solution for calibration to calculate a deviation from the package insert value of the control sample (step S604). Then, the control sample is measured, and a value obtained by correcting a measured value is obtained (step S605) .

<Embodiment 3>

The predicted value $EMF_{HCO3}$ is obtained in step S603 in Embodiment 2 while the selectivity coefficient $K_{Cl,HCO3}$ of the bicarbonate ions is obtained in Embodiment 3.

An operator inputs a concentration value of the bicarbonate ions contained in the control sample via the input unit 123 or the like (step S601). Then, the computer system 200 executes calibration (step S602), and obtains measured values ($EMF_{IS}$, $EMF_L$, and $EMF_H$). Then, the computer system 200 obtains the selectivity coefficient $K_{Cl,HCO3}$ of the bicarbonate ions by inputting the measured values of calibration shown in Table 18 into a function of an algorithm recorded in the storage unit 121 (step S603) .

[Table 18]

| Electrode Number | $EMF_{IS}$ (mV) | $EMF_L$ (mV) | $EMF_H$ (mV) | Slope sensitivity (mV/dec.) |
|---|---|---|---|---|
| 4 | 33.5 | 35.1 | 31.3 | -52.2 |

As a result, the selectivity coefficient of the bicarbonate ions was 0.12. Subsequently, the computer system 200 multiplies, by the obtained selectivity coefficient of the bicarbonate ions, a difference between the concentration of the bicarbonate ions contained in the control sample and the concentrations of the bicarbonate ions contained in the internal standard solution and in the high-concentration standard solution and the low-concentration standard solution for calibration to calculate a deviation from the package insert value of the control sample (step S604). Then, the control sample is measured, and a value obtained by correcting a measured value is obtained (step S605).

The invention is not limited to the above-described embodiments, and includes various modifications. The above-described embodiments have been described in detail to facilitate understanding of the invention, and the invention is not necessarily limited to those including all the configurations described above. A part of a configuration in one embodiment can be replaced with a configuration in another embodiment, and a configuration in one embodiment can also be added to a configuration in another embodiment. A part of a configuration in each embodiment may also be added to, deleted from, or replaced with another configuration.

In Embodiment 1, measured potentials of three types of solutions having the same bicarbonate ion concentration are used as a learning data set. Alternatively, a model may be trained using measured potentials of two types of solutions having the same bicarbonate ion concentration as a learning data set, or a model may be trained using measured potentials of four or more types of solutions as a learning data set. Accuracy is improved as the type of solutions whose measured potentials are to be used in learning increases.

In Embodiment 1, the measured potentials of a plurality of solutions having the same bicarbonate ion concentration are used as a learning data set. Alternatively, a model may be trained by using measured potentials of a plurality of solutions having different bicarbonate ion concentrations as a learning data set. By such training, it is possible to obtain a selectivity coefficient of interfering ions or a value corresponding to a concentration of the interfering ions based on the measured potentials of the plurality of solutions having different bicarbonate ion concentrations.

In Embodiment 1, the learning data set is a combination of measured potentials of a plurality of solutions and a ground truth value. Alternatively, the learning data set may be a combination of a measured potential of one solution containing bicarbonate ions and a ground truth value. By training using such a learning data set, it is possible to obtain a selectivity coefficient of interfering ions or a value corresponding to a concentration of the interfering ions based on a measured value of the one solution.

In Embodiment 1, the trained model is stored in the storage unit 121. Alternatively, the trained model may be stored in an on-premises server or a cloud server that is communicably connected to the electrolyte concentration measuring device 100. In this case, a measured value measured by the electrolyte concentration measuring device 100 is transmitted to the server that stores the trained model via a communication line, and the selectivity coefficient of the interfering ions or the value corresponding to the concentration of the interfering ion is obtained as a return value.

In Embodiment 1, the trained model outputs the selectivity coefficient of the interfering ions or the value corresponding to the concentration of the interfering ions. Alternatively, the invention may not use a trained model, but select a value that approximates a measured value measured by the electrolyte concentration measuring device 100 from data shown in FIG. 4, and obtain a selectivity coefficient associated with the selected data.

Reference Signs List

100: electrolyte concentration measuring device
101: sample container
102: sample aliquoting nozzle
103: sample aliquoting nozzle syringe
104: diluent tank
105: diluent bottle
106: diluent syringe
107: diluent solenoid valve
108: sipper syringe
109: sipper syringe solenoid valve
110: pinch valve
111: sodium ion selective electrode
112: potassium ion selective electrode
113: chloride ion selective electrode
114: reference electrolyte bottle
115: reference electrolyte solenoid valve
116: reference electrode
117: internal standard solution bottle
118: internal standard solution syringe
119: internal standard solution solenoid valve
120: control unit
121: storage unit
122: display unit
123: input unit
200: computer system
201: processor
202: main storage unit
203: auxiliary storage unit
204: input and output interface
205: communication interface
206: bus

**Claims**

1.  An electrolyte concentration measuring device for measuring an ion concentration of an analyte ion contained in a liquid, the electrolyte concentration measuring device comprising:

    an ion selective electrode configured to react with the analyte ion and output a potential corresponding to the ion concentration;
    a reference electrode configured to output a reference potential serving as a reference for the potential; and
    a control unit configured to output the ion concentration of the analyte ion based on a potential difference between the reference electrode and the ion selective electrode, wherein
    the control unit obtains a selectivity coefficient of an interfering ion that influences measurement of a concentration of the analyte ion or a value corresponding to a concentration of the interfering ion from a calculation unit by inputting, into the calculation unit, a measured potential of a solution containing the analyte ion and the interfering ion, the calculation unit being configured to output the selectivity coefficient of the interfering ion or the value corresponding to the concentration of the interfering ion based on the measured potential of the solution containing the analyte ion and the interfering ion.

2. The electrolyte concentration measuring device according to claim 1, wherein
the calculation unit is a trained model that is trained using a plurality of supervised learning data sets in which the measured potential of the solution containing the analyte ion and the interfering ion and the selectivity coefficient of the interfering ion or the value corresponding to the concentration of the interfering ion, which is a ground truth value corresponding to the measured potential, are set.

3. The electrolyte concentration measuring device according to claim 2, wherein
the measured potential in the supervised learning data sets is obtained by measuring a plurality of solutions having a same concentration of the interfering ion and different concentrations of the analyte ion.

4. The electrolyte concentration measuring device according to claim 3, wherein
the value corresponding to the concentration of the interfering ion which is the ground truth value is a measured potential of a solution having a concentration of the interfering ion different from the concentration in the plurality of solutions.

5. The electrolyte concentration measuring device according to claim 1, further comprising:
the calculation unit.

6. The electrolyte concentration measuring device according to claim 1, wherein
the control unit compares a reference value with the selectivity coefficient of the interfering ion or the value corresponding to the concentration of the interfering ion output by the calculation unit, and generates an alarm based on a comparison result between the reference value and the selectivity coefficient of the interfering ion or the value corresponding to the concentration of the interfering ion.

7. The electrolyte concentration measuring device according to claim 1, wherein
the control unit compares a reference value with the selectivity coefficient of the interfering ion or the value corresponding to the concentration of the interfering ion output by the calculation unit, compares a predetermined range with a slope sensitivity calculated based on the measured potential of the solution containing the analyte ion and the interfering ion, and generates an alarm based on a comparison result between the reference value and the selectivity coefficient of the interfering ion or the value corresponding to the concentration of the interfering ion and a comparison result between the slope sensitivity and the predetermined range.

8. The electrolyte concentration measuring device according to claim 1, wherein
the control unit obtains the selectivity coefficient of the interfering ion or the value corresponding to the concentration of the interfering ion from the calculation unit by executing calibration for obtaining a slope sensitivity, and inputting, into the calculation unit, the measured potential of the solution containing the analyte ion and the interfering ion measured in the calibration.

9. The electrolyte concentration measuring device according to claim 1, wherein
the control unit corrects a measured value of a control sample measured for accuracy management of the electrolyte concentration measuring device by using the selectivity coefficient of the interfering ion or the value corresponding to the concentration of the interfering ion obtained from the calculation unit.

10. A method for obtaining a selectivity coefficient for obtaining a selectivity coefficient of an interfering ion that influences measurement of a concentration of an analyte ion contained in a liquid or a value corresponding to a concentration of the interfering ion, the method comprising:

measuring a measured potential of a solution containing the analyte ion and the interfering ion;
inputting the measured potential into a calculation unit configured to output the selectivity coefficient of the interfering ion or the value corresponding to the concentration of the interfering ion based on the measured potential of the solution containing the analyte ion and the interfering ion; and
obtaining the selectivity coefficient of the interfering ion or the value corresponding to the concentration of the interfering ion from the calculation unit.

11. The method for obtaining a selectivity coefficient according to claim 10, further comprising:
    training the calculation unit using a plurality of supervised learning data sets in which the measured potential of the solution containing the analyte ion and the interfering ion and the selectivity coefficient of the interfering ion or the value corresponding to the concentration of the interfering ion, which is a ground truth value corresponding to the measured potential, are set.

12. The method for obtaining a selectivity coefficient according to claim 11, wherein
    the measured potential of the supervised learning data set is obtained by measuring a plurality of solutions having a same concentration of the interfering ion and different concentrations of the analyte ion.

13. The method for obtaining a selectivity coefficient according to claim 12, wherein
    the value corresponding to the concentration of the interfering ion which is the ground truth value is a measured potential of a solution having a concentration of the interfering ion different from the concentration in the plurality of solutions.

14. The method for obtaining a selectivity coefficient according to claim 10, further comprising:

    comparing a reference value with the selectivity coefficient of the interfering ion or the value corresponding to the concentration of the interfering ion output by the calculation unit; and
    generating an alarm based on a comparison result between the reference value and the selectivity coefficient of the interfering ion or the value corresponding to the concentration of the interfering ion.

15. The method for obtaining a selectivity coefficient according to claim 10, further comprising:

    comparing a reference value with the selectivity coefficient of the interfering ion or the value corresponding to the concentration of the interfering ion output by the calculation unit;
    comparing a predetermined range with a slope sensitivity calculated based on the measured potential of the solution containing the analyte ion and the interfering ion; and
    generating an alarm based on a comparison result between the reference value and the selectivity coefficient of the interfering ion or the value corresponding to the concentration of the interfering ion and a comparison result between the slope sensitivity and the predetermined range.

16. The method for obtaining a selectivity coefficient according to claim 10, wherein
    measurement of the measured potential of the solution containing the analyte ion and the interfering ion is executed in calibration for obtaining a slope sensitivity.

17. The method for obtaining a selectivity coefficient according to claim 10, further comprising:
    correcting a measured value of a control sample measured for accuracy management using the selectivity coefficient of the interfering ion or the value corresponding to the concentration of the interfering ion obtained from the calculation unit.

[FIG. 1A]

[FIG. 1B]

[FIG. 2]

**LEARNING PHASE**

INITIAL MODEL — S201

LEARNING

LEARNING DATA SET — S202

FINAL MODEL — S203

**PREDICTION PHASE**

FINAL MODEL — S203

OBJECTIVE DATA — S204

PREDICTION

PREDICTED VALUE — S205

[FIG. 3]

STEP 1: DIVIDE DATA SET INTO TRAINING DATA AND TEST DATA — S301

⇓

STEP 2: STANDARDIZE FEATURE DATA — S302

⇓

STEP 3: SPECIFY INITIAL MODEL — S303

⇓

STEP 4: SPECIFY LOSS FUNCTION — S304

⇓

STEP 5: TRAIN MODEL
USING TRAINING DATA AND LOSS FUNCTION — S305

⇓

STEP 6: EVALUATE MODEL USING TEST DATA — S306

EP 4 481 374 A1

[FIG. 4]

| No. | $EMF_{IS}$ | $EMF_L$ | $EMF_H$ | $EMF_{HCO3}$ | $K_{Cl,HCO3}$ |
|---|---|---|---|---|---|
| 1 | 33.9 | 39.1 | 29.3 | 22.5 | 0.02 |
| 2 | 32.2 | 36.2 | 39.1 | 28.2 | 0.12 |
| 3 | 33.2 | 33.3 | 27.6 | 25.2 | 0.09 |
| 4 | 35.1 | 36.1 | 29.2 | 27.9 | 0.05 |
| 5 | 30.8 | 35.4 | 35.8 | 24.1 | 0.13 |

| No. | $EMF_{IS}$ | $EMF_L$ | $EMF_H$ | $EMF_{HCO3}$ | $K_{Cl,HCO3}$ |
|---|---|---|---|---|---|
| 995 | 33.5 | 37.1 | 25.3 | 22.9 | 0.05 |
| 996 | 35.2 | 36.8 | 39.6 | 29.2 | 0.15 |
| 997 | 31.2 | 38.3 | 22.6 | 35.2 | 0.11 |
| 998 | 35.5 | 36.9 | 29.8 | 27.1 | 0.06 |
| 999 | 30.5 | 32.4 | 32.8 | 29.1 | 0.18 |
| 1000 | 31.6 | 38.2 | 30.6 | 25.1 | 0.07 |

[FIG. 5(a)]

[FIG. 5(b)]

[FIG. 6(a)]

[FIG. 6(b)]

[FIG. 7(a)]

[FIG. 7(b)]

[FIG. 8(a)]

[FIG. 8(b)]

[FIG. 9(a)]

[FIG. 9(b)]

[FIG. 10(a)]

[FIG. 10(b)]

[FIG. 11(a)]

[FIG. 11(b)]

[FIG. 12(a)]

[FIG. 12(b)]

[FIG. 13(a)]

[FIG. 13(b)]

[FIG. 14(a)]

[FIG. 14(b)]

[FIG. 15(a)]

[FIG. 15(b)]

[FIG. 16(a)]

[FIG. 16(b)]

[FIG. 17]

```
                    ┌──────────┐
                    │  START   │
                    └────┬─────┘
                         │
                         ▼
          ┌─────────────────────────┐
          │   EXECUTE CALIBRATION    │──── S401
          └────────────┬────────────┘
                       │
                       ▼
          ┌─────────────────────────┐
          │   OBTAIN SELECTIVITY     │
          │    COEFFICIENT OF        │──── S402
          │   BICARBONATE IONS       │
          │   FROM FINAL MODEL       │
          └────────────┬────────────┘
                       │
                       ▼              S403
              ╱─────────────────╲
             ╱    SELECTIVITY     ╲
            ╱ COEFFICIENT OF BICARBONATE╲      YES
            ╲ IONS IS REFERENCE VALUE  ╱──────────────┐
             ╲      OR MORE?      ╱                    │
              ╲─────────────────╱                      │
                       │ NO                            ▼                S404
                       ▼                       ╱──────────────────╲
                 ┌──────────┐                 ╱  SLOPE SENSITIVITY  ╲     YES
                 │   END    │                 ╲ IS OUTSIDE PREDETERMINED╱────────┐
                 └──────────┘                  ╲      RANGE?        ╱            │
                                                ╲──────────────────╱            │
                                                      │ NO                      │
                                                      ▼          S405           ▼          S406
                                              ┌──────────────┐         ┌──────────────┐
                                              │   MEMBRANE   │         │   MEMBRANE   │
                                              │   FOULING    │         │   PEELING    │
                                              └──────┬───────┘         └──────┬───────┘
                                                     ▼                        ▼
                                               ┌──────────┐            ┌──────────┐
                                               │   END    │            │   END    │
                                               └──────────┘            └──────────┘
```

[FIG. 18]

[FIG. 19]

```
                    ( START )
                        |
        ┌───────────────────────────────┐
        │ INPUT CONCENTRATION           │
        │ OF BICARBONATE IONS           │── S601
        │ OF CONTROL SAMPLE             │
        └───────────────────────────────┘
                        |
        ┌───────────────────────────────┐
        │ EXECUTE CALIBRATION           │── S602
        └───────────────────────────────┘
                        |
        ┌───────────────────────────────┐
        │ OBTAIN SELECTIVITY            │
        │ COEFFICIENT OF                │── S603
        │ BICARBONATE IONS FROM         │
        │ FINAL MODEL                   │
        └───────────────────────────────┘
                        |
        ┌───────────────────────────────┐
        │ CALCULATE DEVIATION FROM      │
        │ PACKAGE INSERT VALUE OF       │── S604
        │ CONTROL SAMPLE                │
        └───────────────────────────────┘
                        |
        ┌───────────────────────────────┐
        │ MEASURE CONTROL               │
        │ SAMPLE AND CORRECT            │── S605
        │ MEASURED VALUE                │
        └───────────────────────────────┘
                        |
                    ( END )
```

## INTERNATIONAL SEARCH REPORT

| | International application No. |
|---|---|
| | **PCT/JP2022/043871** |

**A. CLASSIFICATION OF SUBJECT MATTER**

*G01N 27/26*(2006.01)i; *G01N 27/416*(2006.01)i
FI: G01N27/26 371D; G01N27/26 371F; G01N27/416 351B; G01N27/416 351K; G01N27/26 371A; G01N27/26 371C; G01N27/26 381A

According to International Patent Classification (IPC) or to both national classification and IPC

**B. FIELDS SEARCHED**

Minimum documentation searched (classification system followed by classification symbols)

G01N27/26; G01N27/416

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); Scopus

**C. DOCUMENTS CONSIDERED TO BE RELEVANT**

| Category* | Citation of document, with indication, where appropriate, of the relevant passages | Relevant to claim No. |
|---|---|---|
| X | JP 7-253409 A (KABUSHIKI KAISHA TOSHIBA) 03 October 1995 (1995-10-03) paragraphs [0001]-[0005], [0008], [0025]-[0027], [0029], [0035], [0037], fig. 2, 7 | 1, 5, 8, 10, 16 |
| Y | paragraphs [0001]-[0005], [0008], [0025]-[0027], [0029], [0035], [0037], fig. 2, 7 | 2-4, 11-13 |
| A | entire text, all drawings | 7, 15, 17 |
| X | US 2013/0304395 A1 (NAIDU, Ravendra) 14 November 2013 (2013-11-14) paragraphs [0005], [0006], [0008], [0009], [0011], [0014], [0016]-[0018], [0020], [0022], [0023], [0057], [0072]-[0074], [0114] | 1-2, 5, 10-11 |
| Y | paragraphs [0005], [0006], [0008], [0009], [0011], [0014], [0016]-[0018], [0020], [0022], [0023], [0057], [0072]-[0074], [0114] | 2-4, 6, 9, 11-14, 17 |
| Y | WO 2019/163281 A1 (HITACHI HIGH-TECHNOLOGIES CORP.) 29 August 2019 (2019-08-29) paragraphs [0001], [0002], [0007], [0022], [0047]-[0049], [0065] | 6, 9, 14, 17 |
| A | JP 61-259156 A (HITACHI, LTD.) 17 November 1986 (1986-11-17) p. 1, right column, 2nd paragraph to p. 2, upper left column, 1st paragraph | 7, 15 |

☑ Further documents are listed in the continuation of Box C. ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **24 January 2023** | **07 February 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

International application No.

**PCT/JP2022/043871**

### C. DOCUMENTS CONSIDERED TO BE RELEVANT

| Category* | Citation of document, with indication, where appropriate, of the relevant passages | Relevant to claim No. |
|---|---|---|
| A | JP 2004-219352 A (KABUSHIKI KAISHA TOSHIBA) 05 August 2004 (2004-08-05) paragraphs [0017], [0019] | 7, 15 |
| A | JP 2004-163349 A (HORIBA, LTD.) 10 June 2004 (2004-06-10) paragraphs [0026], [0029] | 7, 15 |
| A | US 2018/0246055 A1 (CRC CARE PTY LTD.) 30 August 2018 (2018-08-30) entire text, all drawings | 1-17 |
| A | SHAMSIPUR, M. et al. Application of artificial neural network to simultaneous potentiometric determination of silver(I), mercury(II) and copper(II) ions by an unmodified carbon paste electrode. Talanta, 2004, vol. 64, no. 3, pp. 590-596, ISSN 1873-3573 entire text, all drawings | 2-4, 11-13 |

Form PCT/ISA/210 (second sheet) (January 2015)

**INTERNATIONAL SEARCH REPORT**
**Information on patent family members**

International application No.

**PCT/JP2022/043871**

| Patent document cited in search report | | | Publication date (day/month/year) | Patent family member(s) | | | Publication date (day/month/year) |
|---|---|---|---|---|---|---|---|
| JP | 7-253409 | A | 03 October 1995 | US | 5580441 | A | |
| | | | | column 1, 1st paragraph to column 2, 4th paragraph, column 2, 7th paragraph, column 7, 4th paragraph to column 8, 1st paragraph, column 8, 3rd paragraph, column 9, 3rd paragraph, column 9, 6th paragraph, fig. 1, 4 | | | |
| | | | | EP | 667522 | A2 | |
| US | 2013/0304395 | A1 | 14 November 2013 | WO | 2012/083371 | A1 | |
| WO | 2019/163281 | A1 | 29 August 2019 | US | 2020/0256821 | A1 | |
| | | | | paragraphs [0001], [0002], [0007], [0031], [0055]-[0057], [0073] | | | |
| | | | | EP | 3757561 | A1 | |
| | | | | CN | 111788479 | A | |
| JP | 61-259156 | A | 17 November 1986 | (Family: none) | | | |
| JP | 2004-219352 | A | 05 August 2004 | (Family: none) | | | |
| JP | 2004-163349 | A | 10 June 2004 | (Family: none) | | | |
| US | 2018/0246055 | A1 | 30 August 2018 | WO | 2016/145481 | A1 | |

Form PCT/ISA/210 (patent family annex) (January 2015)

**REFERENCES CITED IN THE DESCRIPTION**

**Patent documents cited in the description**

- WO 2019163281 A